# EUROPÄISCHE PATENTSCHRIFT

(11) **EP 3 710 424 B1**
(45) Veröffentlichungstag und Bekanntmachung des Hinweises auf die Patenterteilung: **03.01.2024**
(21) Anmeldenummer: 18804565.2
(22) Anmeldetag: 14.11.2018
(51) Int. Cl.: C07C 323/16, C07C 319/18, C09K 15/14, C08K 5/372, C08K 5/375

(54) **VERBINDUNGEN MIT STABILISIERENDER WIRKUNG, VERFAHREN ZU DEREN HERSTELLUNG, ZUSAMMENSETZUNG ENTHALTEND DIESE STABILISIERENDEN VERBINDUNGEN, VERFAHREN ZUR STABILISIERUNG EINER ORGANISCHEN KOMPONENTE SOWIE VERWENDUNG VON STABILISIERENDEN VERBINDUNGEN**
COMPOUNDS HAVING A STABILIZING EFFECT, METHOD FOR PRODUCING SAID COMPOUNDS, COMPOSITION CONTAINING SAID STABILIZING COMPOUNDS, METHOD FOR STABILIZING AN ORGANIC COMPONENT, AND USE OF STABILIZING COMPOUNDS
COMPOSÉS À EFFET STABILISANT, LEUR PROCÉDÉ DE PRÉPARATION, COMPOSITION CONTENANT CES COMPOSÉS STABILISANTS, PROCÉDÉ DE STABILISATION D'UN COMPOSANT ORGANIQUE ET UTILISATION DE COMPOSÉS STABILISANTS

(30) Priorität: 17.11.2017 DE 102017220555
(43) Veröffentlichungstag der Anmeldung: 23.09.2020
(73) Patentinhaber: Fraunhofer-Gesellschaft zur Förderung der angewandten Forschung e.V., 80686 München (DE)
(72) Erfinder: FISCHER, Johannes, 65388 Schlangenbad (DE); METZSCH-ZILLINGEN, Elke, 54597 Steffeln (DE); PFAENDNER, Rudolf, 64668 Rimbach (DE)
(74) Vertreter: Pfenning, Meinig & Partner mbB
(86) Internationale Anmeldenummer: PCT/EP2018/081270
(87) Internationale Veröffentlichungsnummer: WO 2019/096868

(56) Entgegenhaltungen:
- DE-A1- 2 364 126
- DE-A1- 3 639 400
- GB-A- 1 103 145
- US-A- 3 285 855
- US-A- 3 988 363

## Beschreibung

Die vorliegende Verbindung betrifft neuartige Verbindungen mit stabilisierender Wirkung, insbesondere Stabilisierung gegenüber oxidativem thermischen und/oder actinischem Abbau oder Schädigung von organischen Materialien. Die Verbindungen werden durch die nachfolgend angegebene allgemeine Formel I repräsentiert. Die vorliegende Erfindung betrifft zudem ein Verfahren zur Herstellung derartiger Verbindungen, Zusammensetzungen enthaltend diese Verbindung, ein Verfahren zur Stabilisierung von organischen Komponenten mit Hilfe der stabilisierenden Verbindungen sowie Verwendung der stabilisierenden Verbindungen zur Stabilisierung organischer Materialien.

Organische Materialien wie Kunststoffe unterliegen Alterungsvorgängen, die letztendlich zu einem Verlust der erwünschten Eigenschaften wie z.B. der mechanischen Kennwerte führen. Dieser Autoxidation genannte Vorgang führt ausgehend von radikalischen Kettenspaltungen durch mechanochemische Prozesse oder durch UV-Strahlung in Gegenwart von Sauerstoff zu Veränderungen der Polymerkette, wie z.B. im Molekulargewicht oder der Bildung neuer chemischer Gruppen. Um diese Alterung zu verhindern oder zumindest zu verzögern werden deshalb Stabilisatoren eingesetzt. Wichtige Vertreter von Stabilisatoren sind Antioxidantien, die mit den bei der Autoxidation gebildeten Radikalen interferieren und damit den Abbauprozess unterbrechen. Man unterscheidet im Allgemeinen zwischen primären Antioxidantien, die direkt mit Sauerstoff-haltigen freien Radikalen oder C-Radikalen reagieren können und sekundären Antioxidantien, die mit intermediär gebildeten Hydroperoxiden reagieren (s. C. Kröhnke et al. Antioxidants in Ullmann's encyclopedia of industrial chemistry, Wiley-VCH Verlag, Weinheim 2015). Typische Vertreter von primären Antioxidantien sind beispielsweise phenolische Antioxidantien, Amine aber auch Lactone. Klassen von sekundären Antioxidantien sind Phosphorverbindungen wie z.B. Phosphite und Phosphonite, aber auch Organo-Schwefelverbindungen wie z.B. Sulfide und Disulfide. Der gemeinsame Einsatz von primären und sekundären Antioxidantien kann dabei zu einem synergistischen Effekt führen. Bekannt sind z.B. die Kombinationen von phenolischen Antioxidantien mit Phosphiten/Phosphoniten, aber auch die Kombinationen von phenolischen Antioxidantien mit Thioverbindungen (s. z.B. I. Vulic et al. Pol. Degr. Stab. 2002, 78, 27-34). Vor dem Hintergrund einer synergistischen Wirkung ist es deshalb auch wünschenswert, Stabilisatoren zu entwickeln, die beide Funktionen, nämlich die der primären und sekundären Antioxidanswirkung, in einem Molekül aufweisen.

Grundsätzlich sind schwefelhaltige Antioxidantien, d.h. Stabilisatoren, die sowohl eine sterisch gehinderte phenolische Gruppe als auch eine Thiogruppe in einem Molekül beinhalten, bekannt und auch kommerziell teilweise verfügbar. Kommerzielle Produkte weisen dabei beispielsweise die folgenden Strukturen auf (Handelsnamen z.B.: Songnox 4150, Irganox 1081, Irganox 1035, Irganox 1520, Irganox 565, Hostanox OSP 1):

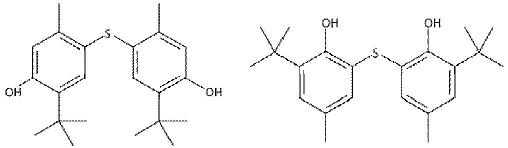

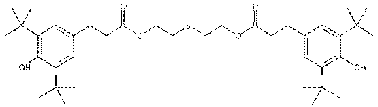

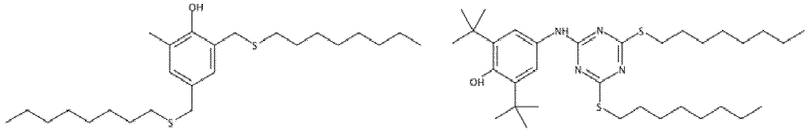

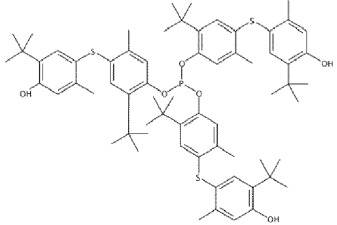

Die Synthese und Verwendung dieser Stabilisatoren ist in zahlreichen Patenten beschrieben, beispielhaft sind erwähnt: DE 23641126, DD 251128, EP 275832, EP 428973, US 3245992, US 3257354

Weiterhin ist bekannt, dass aliphatische Thiogruppen eine geringere Verfärbungstendenz aufweisen als aromatische Thiogruppen (z.B. US 2981717), was im Allgemeinen für eine Langzeitstabilität von organischen Materialien eine erwünschte Eigenschaft ist.

Weitere Stabilisatoren, die sowohl phenolische als auch Thiogruppen beinhalten sind in Form von Isocyanurat enthaltenden Molekülen in den Patentschriften US 4727103, US 4633008 und US 4694102 beschrieben. Die in diesen Patenten erwähnten chemischen Strukturen sind sowohl unterschiedlich zu den erfindungsgemäßen Strukturen als auch nach anderen Verfahren hergestellt.

Bei der Betrachtung der o.a. kommerziellen schwefelhaltigen Antioxidantien fällt auf, dass es offensichtlich keine kommerziellen Produkte gibt, die gleichzeitig eine hohe Konzentration an sterisch gehinderten Phenolen und eine hohe Konzentration von aliphatischen Thiogruppen aufweisen.

Die DE 23 64 126 A1 offenbart ein Verfahren, mit dem verschiedenartige Ester und Amide, die Hydroxyalkylphenylgruppen enthalten, ohne Isolierung von Zwischenprodukten hergestellt werden können.

Die GB 1 103 145 A offenbart ein Verfahren zur Herstellung von organischen Estern, insbesondere von verschiedenen Estern von Hydroxyphenyl(nieder)alkansäuren.

Die US 3 988 363 A offenbart Ester und Amide von 2,4,6-Trialkyl-3-hydroxyphenylalkansäuren und offenbart organische Materialien, die mit diesen Estern und Amiden stabilisiert sind.

Die US 3 285 855 A offenbart eine Stabilisierung von organischem Material, das normalerweise einem oxidativen Verfall unterliegt, insbesondere eine Stabilisierung von Polypropylen mit Verbindungen, die Ester von gehinderten Hydroxybenzoesäuren bzw. Hydroxyphenylalkansäuren sind.

Die DE 36 39 400 A1 offenbart Ester von 3-tert.-Butyl- bzw. 3-tert.-Butyl-5-alkyl-4-hydroxyphenyl-(alkan)-carbonsäuren mit Oxethylaten von monofunktionellen Thiophenolen und Thiolen, Verfahren zu deren Herstellung und deren Verwendung als Stabilisatoren.

Aufgabe der vorliegenden Erfindung war es daher ein neues Verfahren zur Herstellung von schwefelhaltigen Antioxidantien und neue Verbindungen mit ausgezeichneter stabilisierender Wirksamkeit für organische Materialien insbes. für Kunststoffe zur Verfügung zu stellen.

Diese Aufgabe wird mit dem Verfahren gemäß Patentanspruch 1 und den neuartigen Verbindungen gemäß Patentanspruch 8 gelöst. Die Herstellung der entsprechenden Verbindungen wird in Patentanspruch 1 benannt. Gegenstand der vorliegenden Erfindung ist zudem eine Zusammensetzung, enthaltend eine zu stabilisierende organische Komponente sowie eine der erfindungsgemäßen Verbindungen gemäß Patentanspruch 8. Die Erfindung betrifft zudem ein Verfahren zur Stabilisierung einer stabilisierenden organischen Komponente gemäß Patentanspruch 13 sowie Verwendungen der erfindungsgemäßen stabilisierenden Verbindungen gemäß Patentanspruch 14. Die jeweilig abhängigen Patentansprüche betreffen vorteilhafte Ausführungsformen.

Die Erfindung betrifft somit eine Verbindung, die ausgewählt ist aus der Gruppe bestehend aus den nachfolgenden Verbindungen 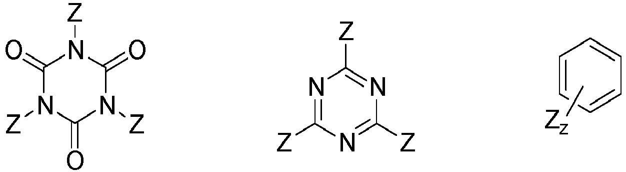 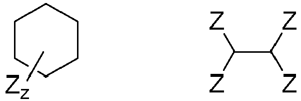 wobei Z die nachfolgende Bedeutung aufweist: 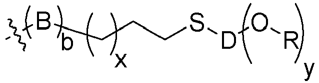 wobei die Variablen B, D, R, b, x, y, z jeweils unabhängig voneinander die nachfolgende Definition aufweisen:
- B: O oder NH,
- D: einen linearen oder verzweigten aliphatischen Rest mit 1 bis 12 Kohlenstoffatomen,
- R: die Bedeutung aufweist: 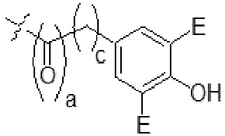
E bei jedem Auftreten gleich oder verschieden ist und einen linear aliphatischen, verzweigt aliphatischen, cycloaliphatischen Alkylrest mit 1 bis 18 Kohlenstoffatomen oder einen aromatischen Rest mit 6 bis 36 Kohlenstoffatomen darstellt,
a 1 oder 0 ist, und
c 0, 1, 2, 3, oder 4 ist,
- b: 0 oder 1,
- x: 0 bis 12,
- y: 1 bis 4,
- z: 2, 3 oder 4.

Es werden neue Stabilisatoren und ein neues Verfahren zur Herstellung der Stabilisatoren vorgeschlagen, die eine einfache Zugänglichkeit für komplexe Stabilisatorstrukturen erlauben und eine hohe Wirksamkeit z.B. in Polymeren aufweisen. Insbesondere der durch das erfindungsgemäße Verfahren hohe Anteil an aliphatischen Schwefelgruppen bei gleichzeitig hoher Anzahl sterisch gehinderter Phenol-Gruppen, führt zu einer geringen Verfärbungstendenz der neuen Verbindungen bei gleichzeitig außerordentlicher Langzeitstabilisierung.

Überraschenderweise hat sich gezeigt, dass die voranstehenden Verbindungen hohes stabilisierendes Potential, insbesondere Stabilisation von organischen Materialien gegen oxidativen thermischen und/oder actinischen Abbau besitzen.

Erfindungsgemäß wird ferner ein Verfahren zur Herstellung einer Verbindung gemäß der allgemeinen Formel I bereitgestellt 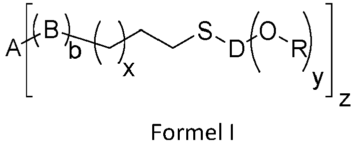 wobei die Variablen A, B, D, R, b, x, y, z jeweils unabhängig voneinander die nachfolgende Definition aufweisen:
- A: einen aromatischen, ungesättigten oder gesättigten Rest,
- B: O oder NH,
- D: einen linearen oder verzweigten aliphatischen Rest mit 1 bis 12 Kohlenstoffatomen,
- R: die Bedeutung aufweist: 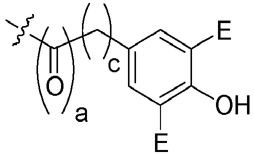 wobei
E bei jedem Auftreten gleich oder verschieden ist und einen linear aliphatischen, verzweigt aliphatischen, cycloaliphatischen Alkylrest mit 1 bis 18 Kohlenstoffatomen oder einen aromatischen Rest mit 6 bis 36 Kohlenstoffatomen darstellt,
a 1 oder 0 ist, und
c 0, 1, 2, 3, oder 4 ist,
- b: 0 oder 1,
- x: 0 bis 12,
- y: 1 bis 4,
- z: 1 bis 6,
wobei bei dem Verfahren eine Verbindung gemäß allgemeiner Formel II 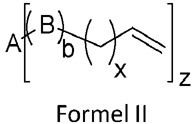 mit einem Thiol gemäß einer der allgemeinen Formeln IIIa oder IIIb 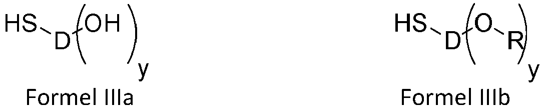 umgesetzt wird, und für den Fall, dass ein Thiol der allgemeinen Formel IIIa verwendet wurde, anschließend eine Umsetzung des durch Reaktion der Verbindungen der Formel II und IIIa erhaltenen Reaktionsproduktes mit einer Verbindung der allgemeinen Formel IV

X-R Formel IV

erfolgt, wobei
- X: eine Abgangsgruppe darstellt.

Eine bevorzugte Ausführungsform sieht vor, dass die Variable A ausgewählt ist aus der Gruppe bestehend aus jeweils z-wertigen Cyanursäureresten, wobei z die im Anspruch 1 angegebene Bedeutung besitzt, Triazinresten, cyclischen aliphatischen Kohlenwasserstoffresten mit 5 bis 36 Kohlenstoffatomen, insbesondere Cyclohexyl, aromatischen Kohlenwasserstoffresten, insbesondere Phenyl, linearen oder verzweigten aliphatischen Kohlenwasserstoffresten mit 2 bis 36 Kohlenstoffatomen.

Erfindungsgemäß weist die Variable R die nachfolgende Bedeutung auf 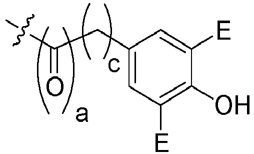 wobei
- E: bei jedem Auftreten gleich oder verschieden ist und einen linear aliphatischen, verzweigt aliphatischen, cycloaliphatischen Alkylrest mit 1 bis 18 Kohlenstoffatomen oder einen aromatischen Rest mit 6 bis 36 Kohlenstoffatomen, insbesondere eine tert-Butyl-Gruppe oder eine Methyl-Gruppe darstellt,
- a: 1 oder 0 ist, und
- c: 0, 1, 2, 3, oder 4 ist.

Bevorzugt sind beide Reste E gleiche Reste. Ebenso ist es jedoch möglich, dass die Reste E verschieden sind.

Insbesondere weisen die Variablen x, y und z jeweils unabhängig voneinander die nachfolgende Bedeutung auf:
- x: 0 oder 1,
- y: 1 oder 2,
- z: 1, 2, 3 oder 4.

Vorzugsweise ist die erfindungsgemäße Verbindung der allgemeinen Formel I ausgewählt aus der Gruppe bestehend aus den nachfolgenden Verbindungen: 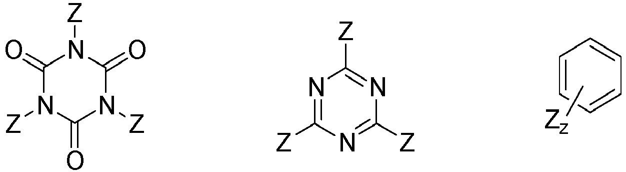 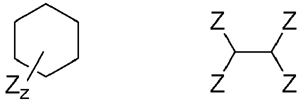 wobei Z die nachfolgende Bedeutung aufweist: 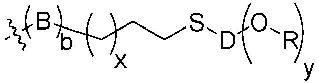 und die Variablen B, D, R, b, x, y und z wie in Anspruch 1 definiert sind.

Die Variable D ist insbesondere -CH₂-.

Besonders bevorzugte Ausführungen der vorliegenden Erfindung sehen vor, dass die erfindungsgemäße Verbindung ausgewählt ist aus der Gruppe der nachfolgenden Verbindungen: 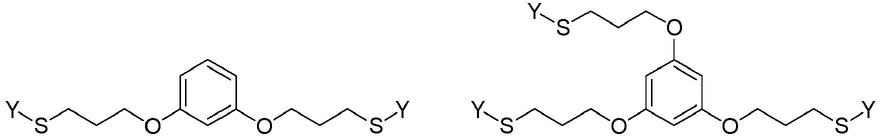 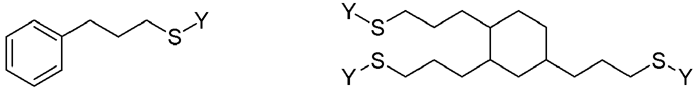 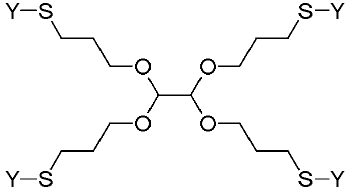 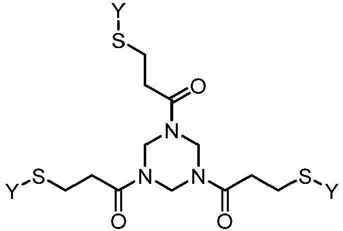 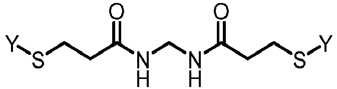 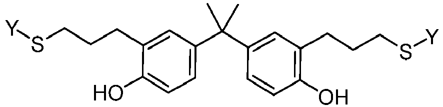 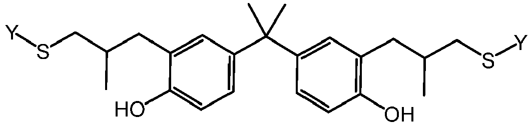 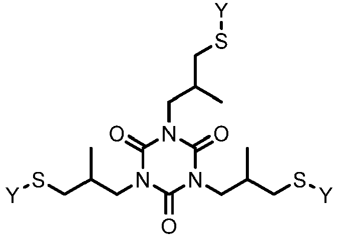 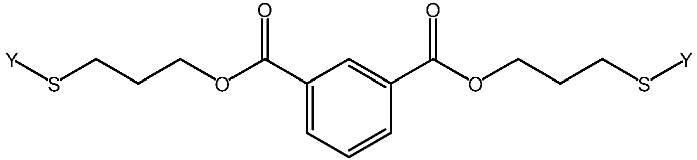 wobei bei jedem Auftreten unabhängig voneinander der Rest Y die nachfolgende Bedeutung aufweist: 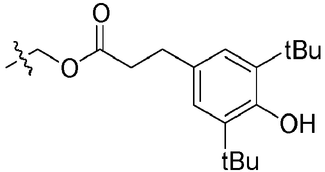 oder

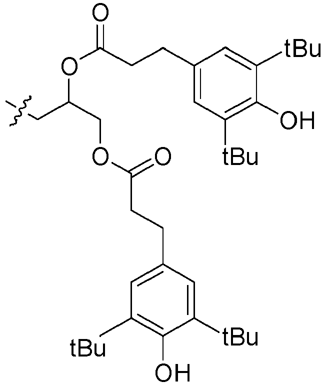

In den voranstehenden Resten kann der tBu-Rest auch ganz oder teilweise durch eine Methylgruppe und/oder Wasserstoff substituiert sein.

Die zuvor erwähnte Abgangsgruppe ist hierbei insbesondere ein Alkoholat, Halogenid, Trifluormethansulfonat, Tosylat, Mesylat, Fluorsulfonat oder Nonaflat.

Die Herstellung der erfindungsgemäßen Verbindungen erfolgt demnach mittels Thiol-en-Reaktion oder Kopplung.

Dabei erfolgt die Reaktion eines Thiols mit reaktiven C-C Doppelbindungen.

Die Thiol-en Kopplung ist eine Reaktion, die z.B. für die Synthese von Beschichtungen, die Herstellung von Folien und für die Modifizierung von Polymeren eingesetzt wird (s. z. B. C. E. Hoyle, C. N. Bowman, Angew. Chem. Int. Ed. 2010, 49, 1540-1573; A. B. Lowe, Polym. Chem. 2010, 1, 17-36). Die Verwendung von Triallylcyanurat, das u.a. als Vorstufe in den erfindungsgemäßen Stabilisatoren verwendet wird, als Ausgangsprodukt für die Thiol-en Kopplung wird von Z. Altintas et al. Chinese Journal of Polymer Science 2015, 6, 850-856 beschrieben. Diese dient als Thioether-funktionalisierter Kettenverlängerer in thermoplastischen Polyurethanen, eine Anwendung als Stabilisator oder Zwischenprodukt für Stabilisatoren ist darin nicht beschrieben oder daraus abzuleiten. Die Verwendung von Diallylphthalat, das u.a. ebenfalls als Vorstufe in den erfindungsgemäßen Stabilisatoren verwendet wird, als Ausgangsprodukt für die Thiol-en Kopplung wird von Herweh et. al. (US4035337) als Inhibierungsagens für (aromatische) Amin-induzierte Vergilbung beschrieben. Eine Molekülkombination und Anwendung mit sterisch gehinderten Phenolen ist darin nicht beschrieben oder daraus abzuleiten. Die Verwendung von Styrol, das u.a. ebenfalls als Vorstufe in den erfindungsgemäßen Stabilisatoren verwendet wird, als Ausgangsprodukt für die Thiol-en Kopplung wird u.a. von Dazzi (US2642373 A) als Fungizid oder von Gluesenkamp (US2617778 A) als Weichmacher für Vinylchlorid-Polymere sowie u.a. von Limnios et. al. Adv. Synth. Catal. 2017, 359, 323-328 als photoinitiierte Alternative zu organokatatalyschen Thiol-Olefin Additionen in möglichen Peptid- und Glucosidmodifizierung beschrieben. Eine Anwendung als Stabilisator oder Zwischenprodukt für Antioxidantien ist auch bei diesen nicht beschrieben oder daraus abzuleiten.

Im Zusammenhang mit Stabilisatoren/Stabilisierung ist die Thiol-en-Reaktion in H. Li et al. Polymer Composites 2015 und W. Wu J. Macromol. Sci. B 2014, 53, 1244-1257 erwähnt. Bezüglich der Reaktionsprinzipien, die auch für die Zwecke der vorliegenden Erfindung Gültigkeit besitzen, wird auf diese Druckschriften verwiesen. Im ersten Fall erfolgt die Synthese eines Silanes über eine Michael-Addition an ein Acrylat mit phenolischer Stabilisatorfunktion. Im zweiten Fall erfolgt zunächst die Reaktion eines polyhydroxylierten Polybutadiens, wobei im Anschluss eine Reaktion mit einem Diisocyanat und einem sterisch gehinderten Phenol erfolgt. Die beiden beschriebenen Reaktionen führen zu anderen chemischen Produkten als die erfindungsgemäßen Stabilisatoren.

Bevorzugt wird die Umsetzung der Verbindung gemäß allgemeiner Formel II mit dem Thiol gemäß einer der allgemeinen Formeln IIIa oder IIIb mit einem Überschuss des Thiols, bezogen auf die ungesättigte Funktion der Verbindung gemäß allgemeiner Formel II durchgeführt.

Zudem betrifft die vorliegende Erfindung eine Zusammensetzung, enthaltend oder bestehend aus mindestens einer zu stabilisierende organische Komponente, sowie mindestens einer erfindungsgemäßen Verbindung wie voranstehend beschrieben.

Stabilisatoren für thermoplastische, elastomere und duromere Kunststoffe insbesondere in Form von Spritzgussteilen, Folien oder Filmen, Beschichtungen oder Lacken, Schäumen, Fasern, Kabeln und Rohren, Profilen, Hohlkörper, Bändchen, Membranen, wie z.B. Geomembranen, oder Klebstoffen ausgebildet sein, die z.B. über Extrusion, Spritzguss, Blasformen, Kalandrieren, Pressverfahren, Spinnprozesse, Rotomoulding oder Streich- und Beschichtungsprozesse hergestellt werden. Anwendung finden die erfindungsgemäßen Zusammensetzungen z.B. für die Elektro- und Elektronikindustrie, Bauindustrie, Transportindustrie (Auto, Flugzeug, Schiff, Bahn), für medizinische Anwendungen, für Haushalts- und Elektrogeräte, Fahrzeugteile, Konsumartikel, Verpackungen, Möbel, Textilien. Ein weiterer Einsatzbereich sind Lacke, Farben und Beschichtungen (Coatings), sowie Öle, Fette wie z.B. Hochleistungsschmierstoffe.

Eine vorteilhafte Ausführungsform der vorliegenden Zusammensetzung sieht vor, dass die zu stabilisierende Komponente ausgewählt ist aus der Gruppe bestehend aus Kunststoffen, Ölen, Schmiermitteln und Fetten.

Geeignete Kunststoffe bzw. Polymere, die in der erfindungsgemäßen Zusammensetzung beinhaltet sein können, sind hierbei insbesondere
a) Polymere aus Olefinen oder Diolefinen wie z.B. Polyethylen (LDPE, LLDPE, VLDPE, ULDPE, MDPE, HDPE, UHMWPE), Metallocen-PE (m-PE), Polypropylen, Polyisobutylen, Poly-4-methyl-penten-1, Polybutadien, Polyisopren, Polycycloocten, Polyalkylen-Kohlenmonoxid-Copolymere, sowie Copolymere in Form von statistischen oder Blockstrukturen wie z.B. Polypropylen-Polyethylen (EP), EPM oder EPDM, Ethylen-Vinylacetat (EVA), Ethylen-Acrylester, wie z.B. Ethylen-Butylacrylat, Ethylen-Acrylsäure und deren Salze (lonomere), sowie Terpolymere wie z.B. Ethylen-Acrylsäure-Glycidylacrylat, Pfropfpolymere wie z.B. Polypropylen-g-Maleinsäureanhydrid, Polypropylen-g-Acrylsäure, Polyethylen-g-Acrylsäure,
b) Polystyrol, Polymethylstyrol, Polyvinylnaphthalin, Styrol-Butadien (SB), Styrol-Butadien-Styrol (SBS), Styrol-Ethylen-Butylen-Styrol (SEBS), Styrol-Ethylen-Propylen-Styrol, Styrolisopren, StyrolIsopren-Styrol (SIS), Styrol-butadien-acrylnitril (ABS), Styrolacrylnitril-acrylat (ASA), Methaccrylat-butadien-styrol (MBS), Methacrylat-acrylonitril-butadien-styrol (MABS), Styrol-Ethylen, Styrol-Maleinsäureanhydrid-Polymere einschl. entsprechender Pfropfcopolymere wie z.B. Styrol auf Butadien, Maleinsäureanhydrid auf SBS oder SEBS, ,
c) halogenenthaltende Polymere wie z.B. Polyvinylchlorid (PVC), Polychloropren und Polyvinylidenchlorid (PVDC), Copolymere aus Vinylchlorid und Vinylidenchlorid oder aus Vinylchlorid und Vinylacetat, chloriertes Polyethylen, Polyvinylidenfluorid,
d) Polymere von ungesättigten Estern wie z.B. Polyacrylate und Polymethacrylate wie Polymethylmethacrylat (PMMA), Polybutylacrylat, Polylaurylacrylat, Polystearylacrylat, Polyacrylnitril, Polyacrylamide, Copolymere wie z.B. Polyacrylnitril-Polyalkylacrylat,
e) Polymere aus ungesättigten Alkoholen und Derivaten, wie z.B. Polyvinylalkohol, Polyvinylacetat, Polyvinylbutyral,
f) Polyacetale, wie z.B. Polyoxymethylen POM) oder Copolymere mit z.B. Butanal,
g) Polyphenylenoxide und Blends mit Polystyrol oder Polyamiden,
h) Polymere von cyclischen Ethern wie z.B. Polyethylenglycol, Polypropylenglycol, Polyethylenoxid, Polypropylenoxid,
i) Polyurethane, aus hydroxyterminierten Polyethern oder Polyestern und aromatischen oder aliphatischen Isocyanaten insbesondere lineare Polyurethane, Polyharnstoffe,
j) Polyamide wie z.B. Polyamid-6, 6.6, 6.10, 4.6, 4.10, 6.12, 12.12, Polyamid 11, Polyamid 12 sowie (teil-)aromatische Polyamide wie z.B. Polyphthalamide, z.B. hergestellt aus Terephthalsäure und/oder Isophthalsäure und aliphatischen Diaminen oder aus aliphatischen Dicarbonsäuren wie z.B. Adipinsäure oder Sebazinsäure und aromatischen Diaminen wie z.B. 1,4- oder 1,3- Diaminobenzol,
k) Polyimide, Polyamid-imide, Polyetherimide, Polyesterimide, Po-ly(ether)ketone, Polysulfone, Polyethersulfone, Polyarylsulfone, Polyphenylensulfid, Polybenzimidazole, Polyhydantoine,
l) Polyester aus aliphatischen oder aromatischen Dicarbonsäuren und Diolen oder aus Hydroxy-Carbonsäuren wie z.B. Polyethylenterephthalat (PET), Polybutylenterephthalat (PBT), Polypropylenterephthalat, Polyethylennaphthylat, Poly-1,4-dimethylolcyclohexanterephthalat, Polyhydroxybenzoat, Polyhydroxynaphthalat, Polymilchsäure,
m) Polycarbonate, Polyestercarbonate, sowie Blends wie z.B. PC/ABS, PC/PBT, PC/PET/PBT,
n) Cellulosederivate wie z.B. Cellulosenitrat, Celluloseacetat, Cellulosepropionat, Cellulosebutyrat
o) nicht-thermoplastischen oder duroplastischen Kunststoffen
p) sowie Mischungen, Kombinationen oder Blends aus zwei oder mehr der zuvor genannten Polymere.

Sofern es sich bei den o.a. Polymeren um Copolymere handelt, können diese in Form von statistischen ("random"), Block- oder "tapered" Strukturen vorliegen oder als Stereoblockcopolymere.

Sofern es sich um stereoreguläre Polymere handelt, können diese in Form von isotaktischen, stereotaktischen, aber auch ataktischen Formen vorliegen.

Ggf. können die Polymere auch vernetzt vorliegen. Eine Vernetzung kann dabei z.B. durch Zugabe von Radikalbildnern oder durch Strahlung wie Elektronenstrahlen, beta- oder gamma-Strahlen während der Verarbeitung oder in einen nachfolgenden Schritt erfolgen.

Die genannten Polymere können dabei nicht nur als Neuware sondern auch in Form von Rezyklaten vorliegen, z.B. als Produktionsabfälle oder aus Wertstoffsammlungen ("post-consumer" Rezyklate).

Weiterhin können die vorliegenden Verbindungen zur Stabilisierung von Kautschuken und Elastomeren eingesetzt werden. Hier kann es sich um Naturkautschuk (NR) oder synthetische Kautschukmaterialien handeln.

Hinsichtlich der enthaltenen Mengen der Verbindung gemäß Formel I ist es bevorzugt, wenn die Zusammensetzung 95,00 bis 99,99 Gew.-Teile, bevorzugt 97,00 bis 99,95 Gew.-Teile, besonders bevorzugt 98,00 bis 99,90 Gew.-Teile mindestens einer zu stabilisierende Komponente, sowie 0,01 bis 5,00 Gew.-Teile, bevorzugt 0,05 bis 3,00 Gew.-Teile, besonders bevorzugt 0,10 bis 2,00 Gew.-Teile, mindestens einer erfindungsgemäßen Verbindung enthält oder hieraus besteht.

Die erfindungsgemäße Zusammensetzung, z.B. auf der Basis von Kunststoffen kann zusätzlich mindestens einen Zusatzstoff beinhalten. Insbesondere ist dieser mindestens eine Zusatzstoff ausgewählt aus der Gruppe bestehend aus UV-Absorbern, Lichtstabilisatoren, Stabilisatoren, Antioxidantien, Hydroxylaminen, Benzofuranonen, Metalldesaktivatoren, Füllstoffdesaktivatoren, Antiozonantien, Nukleierungsmitteln, Schlagzähigkeitsverbesserern, Weichmachern, Gleitmitteln, Rheologiemodifikatoren, Thixotropiemitteln, Kettenverlängerern, Verarbeitungshilfsmitteln, Entformungshilfsmitteln, Flammschutzmitteln, Pigmenten, Farbstoffen, optischen Aufhellern, antimikrobiellen Wirkstoffen, Antistatika, Slipmitteln, Antiblockiermitteln, Kopplungsmitteln, Vernetzungsmitteln, Antivernetzungsmitteln, Hydrophilisierungsmitteln, Hydrophobierungsmitteln, Haftvermittlern, Dispergiermitteln, Abbau-Additiven, Entschäumungshilfsmitteln, Geruchsfängern, Markierungsmitteln, Antifoggingmitteln, Füllstoffen und Verstärkungsstoffen.

Geeignete Lichtstabilisatoren sind beispielsweise Verbindungen auf der Basis von 2-(2'-Hydroxyphenyl)benzotriazolen, 2-Hydroxybenzophenonen, Estern von Benzoesäuren, Acrylaten, Oxamiden und 2-(2-Hydroxyphenyl)-1,3,5-Triazinen.

Geeignete 2-(2'-Hydroxyphenyl)benzotriazole sind beispielsweise 2-(2'-Hydroxy-5'methylphenyl)benzotriazol, 2-(3',5'-Di-*tert*-butyl-2'-hydroxyphenyl)benzotriazol, 2-(5'-*tert*-Butyl-2'-hydroxy-phenyl)benzotriazol, 2-(2'-Hydroxy-5'-(1,1,3,3-tetramethylbutyl)phenyl)benzotriazol, 2-(3',5'-Di-*tert-*butyl-2'-hydroxyphenyl)-5-chlorobenzotriazol, 2-(3'-*tert*-Butyl-2'-hydroxy-5'-methylphenyl-5-chlorobenzotriazol, 2-(3'-sec-Butyl-5'-*tert*-butyl-2'-hydroxyphenyl)benzotriazol, 2-(2'-Hydroxy-4'-octyloxyphenyl)benzotriazol, 2-(3',5'-Di-*tert*-amyl-2'-hydroxyphenyl)benzotriazol, 2-(3',5'-Bis(α,α-dimethylbenzyl)-2'-hydroxyphenyl)benzotriazol, 2-(3'-*tert*-Butyl-2'-hydroxy-5'-(2-octyloxy-carbonylethyl)phenyl)-5-chlorobenzotriazol, 2-(3'-*tert*-Butyl-5'-[2-(2-ethyl-hexyloxy)carbonylethyl]-2'-hydroxyphenyl)-5-chlorobenzotriazol, 2-(3'-*tert-*Butyl-2'-hydroxy-5'-(2-methoxycarbonylethyl)phenyl)-5-chlorobenzotriazol, 2-(3'-*tert*-Butyl-2'-hydroxy-5'-(2-methoxycarbonylethyl)phenyl)benzotriazol, 2-(3'-*tert*-Butyl-2'-hydroxy-5'-(2-octyloxycarbonylethyl)phenyl)benzotriazol, 2-(3'-*tert*-Butyl-5'-[2-(2-ethylhexyloxy)carbonylethyl]-2'-hydroxyphenyl)benzotriazol, 2-(3'-Dodecyl-2'-hydroxy-5'-methylphenyl)benzotriazol, 2-(3'-*tert-*Butyl-2'-hydroxy-5'-(2-isooctyloxycarbonylethyl)phenylbenzotriazol, 2,2'-Methylenbis[4-(1,1,3,3-tetramethylbutyl)-6-benzotriazol-2-ylphenol]; das Produkt der Umesterung von 2-[3'-*tert*-Butyl-5'-(2-methoxycarbonylethyl)-2'-hydroxyphenyl]-2H-benzotriazol mit Polyethylenglycol 300; [R-CH₂CH₂- 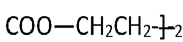 , wobei R = 3'-*tert*-Butyl-4'-hydroxy-5'-2H-benzotriazol-2-ylphenyl, 2-[2'-Hydroxy-3'-(α,α-dimethylbenzyl)-5'-(1,1,3,3-tetramethylbutyl)-phenyl]benzotriazol, 2-[2'-hydroxy-3'-(1,1,3,3-tetramethylbutyl)-5'-(α,α-dimethylbenzyl)phenyl]benzotriazol.

Geeignete 2-Hydroxybenzophenone sind beispielsweise 4-Hydroxy-, 4-Methoxy-, 4-Octyloxy-, 4-Decyloxy- 4-Dodecyloxy, 4-Benzyloxy, 4,2',4'-Trihydroxy- und 2'-Hydroxy-4,4'-dimethyoxy-Derivate der 2-Hydroxybenzophenone.

Geeignete Acrylate sind beispielsweise Ethyl-α-cyano-β,β-diphenylacrylat, Isooctyl-α-cyano-β,β-diphenylacrylat, Methyl-α-carbomethoxycinnamat, Methyl-α-cyano-β-methyl-p-methoxycinnamat, Butyl-α-cyano-β-methyl-p-methoxycinnamat, Methyl-α-carbomethoxy-p-methoxycinnamat und N-(β-carbomethoxy-β-cyanovinyl)-2-methylindolin.

Geeignete Ester von Benzoesäuren sind beispielsweise 4-*tert*-Butylphenylsalicylat, Phenylsalicylat, Octylphenylsalicylat, Dibenzoylresorcinol, Bis(4-*tert-*butylbenzoyl)resorcinol, Benzoylresorcinol, 2,4-Di-*tert*-butylphenyl-3,5-di-*tert-*butyl-4-hydroxybenzoat, Hexadecyl-3,5-di-*tert*-butyl-4-hydroxybenzoat, Octadecyl-3,5-di-*tert*-butyl-4-hydroxybenzoat, 2-Methyl-4,6-di-*tert*-butylphenyl-3,5-di-*tert*-butyl-4-hydroxybenzoat.

Geeignete Oxamide sind beispielsweise 4,4'-Dioctyloxyoxanilid, 2,2'-diethoxyoxanilid, 2,2'-Dioctyloxy-5,5'-di-*tert*-butoxanilid, 2,2'-didodecyloxy-5,5'-di-*tert-*butoxanilid, 2-Ethoxy-2'-ethyloxanilid, N,N'-Bis(3-dimethylaminopropyl)-oxamid, 2-Ethoxy-5-*tert*-butyl-2'-ethoxanilid und seine Mischungen mit 2-Ethoxy-2'-ethyl-5,4'-di-*tert*-butoxanilid, Mischungen von o- und p-Methoxydisubstituierten Oxaniliden und Mischungen von o- und p-Ethoxydisubstituierten Oxaniliden.

Geeignete 2-(2-Hydroxyphenyl)-1,3,5-Triazine sind beispielsweise 2,4,6-Tris(2-hydroxy-4-octyloxyphenyl)-1,3,5-triazin, 2-(2-Hydroxy-4-octyloxyphenyl)-4,6-bis(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl-1,3,5-triazin, 2-(2-Hydroxy-4-dodecyloxyphenyl)-4,6-bis(2,4-dimethylphenyl)-1,3,5-triazin, 2-(2-Hydroxy-4-tridecyloxyphenyl)-4,6-bis(2,4-dimethylphenyl)-1,3,5-triazin, 2-[2-Hydroxy-4-(2-hydroxy-3-butyloxypropoxy)-phenyl]-4,6-bis(2,4-dimethyl)-1,3,5-triazin, 2-[2-Hydroxy-4-(2-hydroxy-3-octyl-oxypropyloxy)phenyl]-4,6-bis(2,4-dimethyl)-1,3,5-triazin, 2-[4-(Dodecyloxy/- Tridecyloxy-2-hydroxypropoxy)-2-hydroxyphenyl]-4,6-bis(2,4-dimethylphenyl)-1,3,5-triazin, 2-[2-Hydroxy-4-(2-hydroxy-3-dodecyloxypropoxy)-phenyl]-4,6-bis(2,4-dimethylphenyl-1,3,5-triazin, 2-(2-Hydroxy-4-hexyloxy)-phenyl-4,6-diphenyl-1,3,5-triazin, 2-(2-Hydroxy-4-methoxyphenyl)-4,6-diphenyl-1,3,5-triazin, 2,4,6-Tris[2-hydroxy-4-(3-butoxy-2-hydroxypropoxy)-phenyl]-1,3,5-triazin, 2-(2-Hydroxyphenyl)-4-(4-methoxyphenyl)-6-phenyl-1,3,5-triazin, 2-{2-Hydroxy-4-[3-(2-ethylhexyl-1-oxy)-2-hydroxypropyloxy]-phenyl}-4,6-bis(2,4-dimethylphenyl-1,3,5-triazin.

Geeignete Metalldesaktivatoren sind beispielsweise N,N'-Diphenyloxamid, N-Salicylal-N'-salicyloylhydrazin, N,N'-Bis(salicyloyl)hydrazin, N,N'-Bis(3,5-di-tert-butyl-4-hydroxyphenylpropionyl)hydrazin, 3-Salicyloylamino-1,2,4-triazol, Bis(benzyliden)oxalyldihydrazid, Oxanilid, Isophthaloyldihydrazid, Sebacoylbisphenylhydrazid, N,N'-Diacetyladipoyldihydrazid, N,N'-Bis(salicyloyl)oxylyldi-hydrazid, N,N'-Bis(salicyloyl)thiopropionyldihydrazid.
Geeignete phenolische Antioxidantien sind beispielsweise: Alkylierte Monophenole, wie z.B. 2,6-Di-*tert*-butyl-4-methylphenol, 2-*tert-*Butyl-4,6-dimethylphenol, 2,6-Di-*tert*-butyl-4-ethylphenol, 2,6-Di-*tert*-butyl-4-n-butylphenol, 2,6-Di-*tert*-butyl-4-isobutylphenol, 2,6-Dicyclopentyl-4-methylphenol, 2-(α-Methylcyclohexyl)-4,6-dimethylphenol, 2,6-Dioctadecyl-4-methylphenol, 2,4,6-Tricyclohexylphenol, 2,6-Di-*tert*-butyl-4-methoxymethyl-phenol, lineare oder verzweigte Nonylphenole, wie z.B. 2,6-Dinonyl-4-methylphenol, 2,4-Dimethyl-6-(1'-methylundec-1'-yl)phenol, 2,4-Dimethyl-6-(1'-methylheptadec-1'-yl)phenol, 2,4-Dimethyl-6-(1'-methyltridec-1'-yl)phenol und Mischungen hiervon;
Alkylthiomethylphenole, wie z.B. 2,4-Dioctylthiomethyl-6-*tert*-butylphenol, 2,4-Dioctylthiomethyl-6-methylphenol, 2,4-Dioctylthiomethyl-6-ethylphenol, 2,6-Didodecylthiomethyl-4-nonylphenol;
Hydrochinone und alkylierte Hydrochinone, wie z.B. 2,6-Di-*tert*-butyl-4-methyoxyphenol, 2,5-Di-*tert*-butylhydrochinon, 2,5-Di-*tert*-amylhydrochinon, 2,6-Diphenyl-4-octadecyloxyphenol, 2,6-Di-*tert*-butylhydrochinon, 2,5-Di-*tert-*butyl-4-hydroxyanisol, 3,5-Di-*tert*-butyl-4-hydroxyanisol, 3,5-Di-*tert*-butyl-4-hydroxyphenylstearat, Bis(3,5-di-*tert*-butyl-4-hydroxylphenyl)adipat; Tocopherole, wie z.B. α-, β-, γ-, δ-Tocopherol und Mischungen aus diesen (Vitamin E);
Hydroxylierte Thiodiphenylether, wie z.B. 2,2'-Thiobis(6-*tert*-butyl-4-methylphenol), 2,2'-Thiobis(4-octylphenol), 4,4'-Thiobis(6-*tert*-butyl-3-methylphenol), 4,4'-Thiobis(6-*tert*-butyl-2-methylphenol), 4,4'-Thiobis(3,6-di-*sec-*amylphenol), 4,4'-Bis(2,6-dimethyl-4-hydroxyphenyl)disulfid; Alkylidenbisphenole, wie z.B. 2,2'Methylenbis(6-*tert*-butyl-4-methylphenol), 2,2'-Methylenbis(6-*tert*-butyl-4-ethylphenol), 2,2'-Methylenbis[4-methyl-6-(α-methylcyclohexyl)phenol], 2,2'-Methylenbis(4-methyl-6-cyclhexylphenol), 2,2'-Methylenbis(6-nonyl-4-methylphenol), 2,2'-Methylenbis(4,6-di-*tert*-butylphenol), 2,2'-Ethylidenbis(4,6-di-*tert*-butylphenol), 2,2'-Ethylidenbis(6-*tert-*butyl-4-isobutylphenol), 2,2'-Methylenbis[6-(α-methylbenzyl)-4-nonylphenol], 2,2'-Methylenbis[6-(α,α-dimethylbenzyl)-4-nonylphenol], 4,4'-Methylenbis-(2,6-di-*tert*-butylphenol, 4,4'-Methylenbis(6-*tert*-butyl-2-methylphenol), 1,1-bis(5-*tert*-butyl-4-hydroxy-2-methylphenyl)butan, 2,6-Bis(3-*tert*-butyl-5-methyl-2-hydroxybenzyl)-4-methylphenol, 1,1,3-Tris(5-*tert*-butyl-4-hydroxy-2-methylphenyl)butan, 1,1-bis(5-*tert*-butyl-4-hydroxy-2-methylphenyl)-3-n-dodecylmercaptobutan, Ethylenglycol-bis[3,3-bis(3'-*tert*-butyl-4'-hydroxy-phenyl)butyrat], Bis(3-*tert*-butyl-4-hydroxy-5-methylphenyl)dicyclopentadien, Bis[2-(3'-*tert*-butyl-2'-hydroxy-5'-methylbenzyl)-6-*tert*-butyl-4-methylphenyl]terephthalat, 1,1-Bis-(3,5-dimethyl-2-hydroxyphenyl)butan, 2,2-Bis(3,5-di-*tert*-butyl-4-hydroxyphenyl)propan, 2,2-Bis-(5-*tert*-butyl-4-hydroxy-2-methylphenyl)-4-n-dodecylmercaptobutan, 1,1,5,5-Tetra(5-*tert*-butyl-4-hydroxy-2-methylphenyl)pentan;
O-, N- und S-Benzyl-Verbindungen, wie z.B. 3,5,3',5'-Tetra-*tert*-butyl-4,4'-dihydroxydibenzylether, Octadecyl-4-hydroxy-3,5-dimethylbenzylmercapto-acetat, Tridecyl-4-hydroxy-3,5-di-*tert*-butylbenzylmercaptoacetat, Tris(3,5-di-*tert*-butyl-4-hydroxybenzyl)amin, , Bis(4-*tert*-butyl-3-hydroxy-2,6-dimethylbenzyl)dithioterephthalat, Bis(3,5-di-*tert*-butyl-4-hydroxybenzyl)sulfid, Isooctyl-3,5-di-*tert*-butyl-4-hydroxybenzylmercaptoacetat;
Hydroxybenzylierte Malonate, wie z.B. Dioctadecyl-2,2-bis(3,5-di-*tert*-butyl-2-hydroxybenzyl)malonat, Dioctadecyl-2-(3-*tert*-butyl-4-hydroxy-5-methylbenzyl)malonat, Didodecylmercaptoethyl-2,2-bis(3,5-di-*tert*-butyl-4-hydroxybenzyl)malonat, Bis[4-(1,1,3,3-tetramethylbutyl)phenyl]-2,2-bis(3,5-di-*tert-*butyl-4-hydroxybenzyl)malonat;
Aromatische Hydroxybenzylverbindungen, wie z.B. 1,3,5-Tris(3,5-di-*tert*-butyl-4-hydroxybenzyl)-2,4,6-trimethylbenzol, 1,4-Bis(3,5-di-*tert*-butyl-4-hydroxybenzyl)-2,3,5,6-tetramethylbenzol, 2,4,6-Tris(3,5-di-*tert*-butyl-4-hydroxybenzyl)phenol;
Triazinverbindungen, wie z.B. 2,4-Bis(octylmercapto)-6-(3,5-di-*tert*-butyl-4-hydroxyanilino)-1,3,5-triazin, 2-Octylmercapto-4,6-bis(3,5-di-*tert*-butyl-4-hydroxyanilino)-1,3,5-triazin, 2-Octylmercapto-4,6-bis(3,5-di-*tert*-butyl-4-hydroxyphenoxy)-1,3,5-triazin, 2,4,6-Tris(3,5-di-*tert*-butyl-4-hydroxyphenoxy)-1,2,3-triazin, 1,3,5-Tris(3,5-di-*tert*-butyl-4-hydroxybenzyl)isocyanurat, 1,3,5-Tris(4-*tert*-butyl-3-hydroxy-2,6-dimethylbenzyl)isocyanurat, 2,4,6-Tris(3,5-di-*tert*-butyl-4-hydroxphenylethyl)-1,3,5-triazin, 1,3,5-Tris(3,5-di-*tert*-butyl-4-hydroyphenylpropionyl)hexahydro-1,3,5-triazin, 1,3,5-Tris(3,5-dicyclohexyl-4-hydroxybenzyl)isocyanurat;
Benzylphosphonate, wie z.B. Dimethyl-2,5-di-*tert*-butyl-4-hydroxybenzyl-phosphonat, Dietyhl-3,5-di-*tert*-butyl-4-hydroxybenzylphosphonat, Di-octadecyl-3,5-di-*tert*-butyl-4-hydroxybenzylphosphonat, Dioctadecyl-5-*tert-*butyl-4-hydroxy-3-methylbenzylphosphonat, das Calciumsalz des Monoethylesters der 3,5-Di-*tert*-butyl-4-hydroxybenzylphosphonsäure; Acylaminophenole, wie z.B. 4-Hydroxylauranilid, 4-Hydroxystearanilid, Octyl-N-(3,5-di-*tert*-butyl-4-hydroxyphenyl)carbamat;
Ester der β-(3,5-Di-*tert*-butyl-4-hydroxyphenyl)propionsäure mit ein- oder mehrwertigen Alkoholen, z.B. Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythritol, Tris(hydroxyethyl)isocyanurat, N,N'-Bis(hydroxyethyl)oxamid, 3-Thiaundecanol, 3-Thiapentadecanol, Trimethylhexandiol, Trimethylolpropan, 4-Hydroxymethyl-1-phospha-2,6,7-trioxabicyclo[2.2.2]octan;
Ester der β-(5-*tert*-Butyl-4-hydroxy-3-methylphenyl)propionsäure mit ein- oder mehrwertigen Alkoholen, z.B. Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythritol, Tris(hydroxyethyl)isocyanurat, N,N'-bis(hydroxyethyl)oxamid, 3-Thiaundecanol, 3-Thiapentadecanol, Trimethylhexandiol, Trimethylolpropan, 4-Hydroxymethyl-1-phospha-2,6,7-trioxabicyclo[2.2.2]octan, 3,9-Bis[2-{3-(3-*tert*-butyl-4-hydroxy-5-methylphenyl)propionyloxy}-1,1-dimethylethyl]-2,4,8,10-tetraoxaspiro[5.5]undecan;
Ester der β-(3,5-Dicyclohexyl-4-hydroxyphenyl)propionsäure mit ein- oder mehrwertigen Alkoholen, z.B. Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythritol, Tris-(hydroxyethyl)isocyanurat, N,N'-bis(hydroxyethyl)oxamid, 3-Thiaundecanol, 3-Thiapentadecanol, Trimethylhexandiol, Trimethylolpropan, 4-Hydroxymethyl-1-phospha-2,6,7-trioxabicyclo[2.2.2]octan;
Ester der (3,5-Di-*tert*-butyl-4-hydroxyphenyl)essigsäure mit ein- oder mehrwertigen Alkoholen, z.B. Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythritol, Tris(hydroxyethyl)isocyanurat, N,N'-bis(hydroxyethyl)oxamid, 3-Thiaundecanol, 3-Thiapentadecanol, Trimethylhexandiol, Trimethylolpropan, 4-Hydroxymethyl-1-phospha-2,6,7-trioxabicyclo[2.2.2]octan;
Amide der β-(3,5-Di-*tert*-butyl-4-hydroxyphenyl)propionsäure, wie z.B. N,N'-Bis(3,5-di-*tert*-butyl-4-hydroxyphenylpropionyl)hexamethylendiamid, N,N'-Bis(3,5-di-*tert*-butyl-4-hydroxyphenylpropionyl)hexamethylendiamid, N,N'-Bis(3,5-di-*tert*-butyl-4-hydroxyphenylpropionyl)hexamethylendiamid, N,N'-Bis(3,5-di-*tert*-butyl-4-hydroxyphenylpropionyl)hydrazid, N,N'-Bis[2-(3-[3,5-di-*tert*-butyl-4-hydroxyphenyl]propionyloxy)ethyl]oxamid (Naugard^{®}XL-1, vertrieben durch Uniroyal);
Ascorbinsäure (Vitamin C).

### Besonders bevorzugte phenolische Antioxidantien sind:

Octadecyl-3-(3,5-di-*tert*-butyl-4-hydroxyphenyl)propionat, Pentaerythritol-tetrakis[3-(3,5-di-*tert*-butyl-4-hydroxyphenyl)propionat, Tris(3,5-di-*tert*-butyl-4-hydroxyphenyl)isocyanurat, 1,3,5-Trimethyl-2,4,6-tris(3,5-di-*tert*-butyl-4-hydroxyphenyl)isocyanurat, 1,3,5-Trimethyl-2,4,6-tris(3,5-di-*tert*-butyl-4-hydroxybenzyl)benzol, Triethylenglycol-bis[3-(3-*tert*-butyl-4-hydroxy-5-methylphenyl)propionat, N,N'-Hexan-1,6-diyl-bis[3-(3,5-di-*tert*-butyl-4-hydroxyphenyl)propionsäureamid.

### Geeignete Phosphite/Phosphonite sind beispielsweise:

Triphenylphosphit, Diphenylalkylphosphite, Phenyldialkylphosphite, Tri(nonylphenyl)phosphit, Trilaurylphosphite, Trioctadecylphosphit, Distearylpentaerythritoldiphosphit, Tris-(2,4-di-*tert*-butylphenyl)phosphit, Diisodecylpentaerythritoldiphosphit, Bis(2,4-di-*tert*-butylphenyl)pentaerythritoldiphosphit, Bis(2,4-di-cumylphenyl)pentaerythritoldiphosphit, Bis(2,6-di-*tert*-butyl-4-methylphenyl)pentaerythritoldiphosphit, Diisodecyloxypentaerythritoldiphosphit, Bis(2,4-di-*tert*-butyl-6-methylphenyl)pentaerythritoldiphosphit, Bis-(2,4,6-tris(*tert*-butylphenyl)pentaerythritoldiphosphit, Tristearylsorbitoltriphosphit, Tetrakis(2,4-di-*tert*-butylphenyl)-4,4'-biphenylendiphosphonit, 6-Isooctyloxy-2,4,8,10-tetra-*tert*-butyl-12H-dibenz[d,g]-1,3,2-dioxaphosphocin, Bis(2,4-di-*tert*-butyl-6-methylphenyl)methylphosphit, Bis(2,4-di-*tert*-butyl-6-methylphenyl)ethylphosphit, 6-Fluoro-2,4,8,10-tetra-*tert*-butyl-12-methyl-di-benz[d,g]-1,3,2-dioxaphosphocin, 2,2'2"-Nitrilo[triethyltris(3,3",5,5'-tetra-*tert*-butyl-1,1'-biphenyl-2,2'-diyl)phosphit], 2-Ethylhexyl(3,3',5,5'-tetra-*tert-*butyl-1,1'-biphenyl-2,2'-diyl))phosphit, 5-Butyl-5-ethyl-2-(2,4,6-tri-*tert*-butyl-phenoxy)-1,3,2-dioxaphosphiran.

Besonders bevorzugte Phosphite/Phosphonite sind: 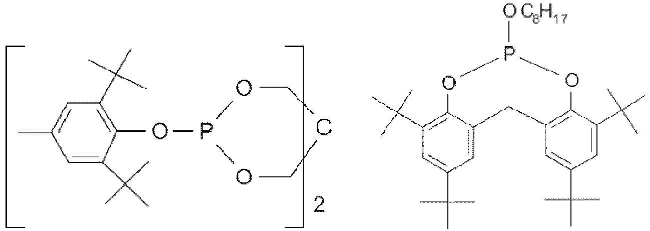 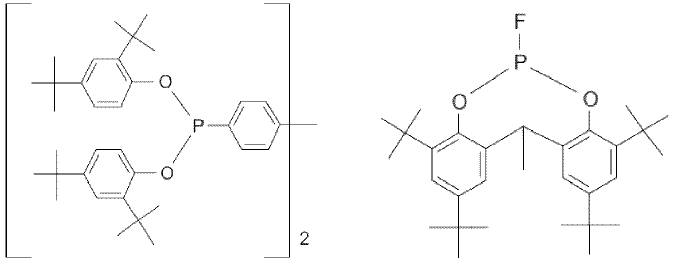 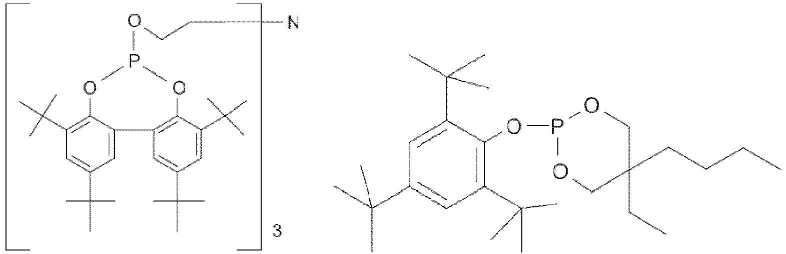 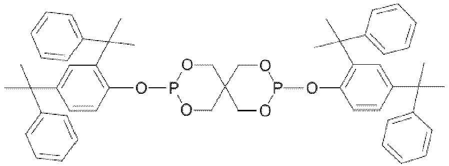 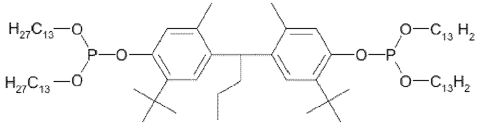 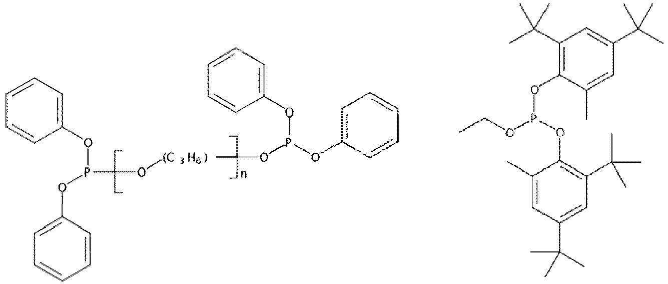 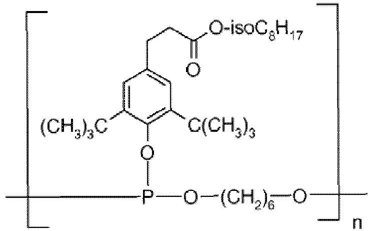 wobei n zwischen 1 und 100 liegen kann.

Weitere geeignete Stabilisatoren sind aminische Antioxidantien.

### Geeignete aminische Antioxidantien sind beispielsweise:

N,N'-Di-isopropyl-p-phenylendiamin, N,N'-Di-sec-butyl-p-phenylendiamin, N,N'-Bis(1,4-dimethylpentyl)-p-phenylendiamin, N,N'-Bis(1-ethyl-3-methylpentyl)-p-phenylendiamin, N,N'-Bis(1-methylheptyl)-p-phenylendiamin, N,N'-Dicyclohexyl-p-phenylendiamin, N,N'-Diphenyl-p-phenylendiamin, N,N'-Bis(2-naphthyl)-p-phenylendiamin, N-Isopropyl-N'-phenyl-p-phenylendiamin, N-(1,3-Dimethylbutyl)-N'-phenyl-p-phenylen-diamin, N-(1-Methylheptyl)-N'-phenyl-p-phenylendiamin, N-Cyclohexyl-N'-phenyl-p-phenylendiamin, 4-(p-Toluolsulfamoyl)diphenylamin, N,N'-Dimethyl-N,N'-di-*sec*-butyl-p-phenylendiamin, Diphenylamin, N-Allyldiphenylamin, 4-Isopropoxydiphenylamin, N-Phenyl-1-naphthylamin, N-(4-tert-Octylphenyl)-1-naphthylamin, N-Phenyl-2-naphthylamin, octyliertes Diphenylamin, z.B. p,p'-Di-tert-octyldiphenylamin, 4-n-Butylaminophenol, 4-Butyrylaminophenol, 4-Nonanoylaminophenol, 4-Dodecanoylaminophenol, 4-Octadecanoylamino-phenol, Bis(4-methoxyphenyl)amin, 2,6-Di-tert-butyl-4-dimethylaminomethyl-phenol, 2,4'-Diaminodiphenylmethan, 4,4'-Diaminodiphenylmethan, N,N,N',N'-Tetramethyl-4,4'-diaminodiphenylmethan, 1,2-Bis[(2-methyl-phenyl)amino]ethan, 1,2-Bis(phenylamino)propan, (o-Tolyl)biguanid, Bis[4-(1',3'-dimethylbutyl)-phenyl]amin, *tert*-octyliertes N-Phenyl-1-naphthylamin, ein Gemisch aus mono- und dialkylierten *tert*-Butyl/*tert*-Octyldiphenylaminen, ein Gemisch aus mono- und dialkylierten Nonyldiphenylaminen, ein Gemisch aus mono- und dialkylierten Dodecyldiphenylaminen, ein Gemisch aus mono- und dialkylierten Isopropyl/Isohexyl-diphenylaminen, ein Gemisch aus mono- und dialkylierten *tert*-Butyldiphenylaminen, 2,3-Dihydro-3,3-dimethyl-4H-1,4-benzothiazin, Phenothiazin, ein Gemisch aus mono- und dialkylierten *tert-*Butyl/*tert*-Octylphenothiazinen, ein Gemisch aus mono- und dialkylierten *tert*-Octylphenothiazinen, N-Allylphenothiazin, N,N,N',N'-Tetraphenyl-1,4-diaminobut-2-en sowie Mischungen oder Kombinationen hiervon.

Bevorzugte aminische Antioxidantien sind: N,N'-Di-isopropyl-p-phenylendiamin, N,N'-Di-*sec*-butyl-p-phenylendiamin, N,N'-Bis(1,4-dimethylpentyl)-p-phenylendiamin, N,N'-Bis(1-ethyl-3-methylpentyl)-p-phenylendiamin, N,N'-Bis(1-methylheptyl)-p-phenylendiamin, N,N'-Dicyclohexyl-p-phenylendiamin, N,N'-Diphenyl-p-phenylendiamin, N,N'-Bis(2-naphthyl)-p-phenylendiamin, N-Isopropyl-N'-phenyl-p-phenylendiamin, N-(1,3-Dimethylbutyl)-N'-phenyl-p-phenylen-diamin, N-(1-Methylheptyl)-N'-phenyl-p-phenylendiamin, N-Cyclohexyl-N'-phenyl-p-phenylendiamin

Weitere bevorzugte aminische Antioxidantien sind Hydroxylamine bzw. N-oxide (Nitrone), wie z.B. N,N-Dialkylhydroxylamine, N,N-Dibenzylhydroxyl-amin, N,N-Dilaurylhydroxylamin, N,N-Distearylhydroxylamin, N-Benzyl-α-phenylnitron, N-Octadecyl-α-hexadecylnitron, sowie Genox EP (Addivant) gemäß der Formel: 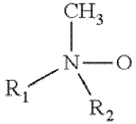 R₁, R₂ = C₁₄-C₂₄ alkyl

Weitere geeignete Stabilisatoren sind Thiosynergisten.

Geeignete Thiosynergisten sind beispielsweise Distearylthiodipropionat, Dilaurylthiodipropionat; Ditridecyldithiopropionat, Ditetradecylthiodipropionat, 3-(dodecylthio)-, 1,1'-[2,2-bis[[3-(dodecylthio)-1-oxopropoxy]methyl]-1,3-propanediyl]propansäureester.

Weitere geeignete Stabilisatoren insbesondere für Polyamide sind Kupfersalze wie z.B. Kupfer-(I)-iodid, Kupfer-(I)-bromid oder Kupferkomplexe wie z.B. Triphenylphosphin-Kupfer-(I)-Komplexe.

Weitere geeignete Stabilisatoren sind Benzofuranone und Indolinone wie z.B. 3-(4-(2-acetoxyethoxy)phenyl]-5,7-di-tert-butyl-benzofuran-2-on, 5,7-di-tert-butyl-3-[4-(2-stearoyloxyethoxy)phenyl]benzofuran-2-on, 3,3'-bis[5,7-di-tert-butyl-3-(4-(2-hydroxyethoxy]phenyl)benzofuran-2-on), 5,7-di-tert-butyl-3-(4-ethoxyphenyl)benzofuran-2-on, 3-(4-acetoxy-3,5-dimethylphenyl)-5,7-di-tert-butyl-benzofuran-2-on, 3-(3,5-dimethyl-4-pivaloyloxyphenyl )-5,7-di-tert-butyl-benzofuran-2-on, 3-(3,4-dimethylphenyl)-5,7-di-tert-butyl-benzo-furan-2-on, 3-(2,3-di methylphenyl)-5,7-di-tert-butyl-benzofuran-2-on.

Geeignete gehinderte Amine sind beispielsweise 1,1-Bis(2,2,6,6-tetramethyl-4-piperidyl)succinat, Bis(1,2,2,6,6-pentamethyl-4-piperidyl)sebazat, Bis(1-octyloxy-2,2,6,6-tetramethyl-4-piperidyl)sebazat, Bis(1,2,2,6,6-pentamethyl-4-piperidyl)-n-butyl-3,5-di-tert-butyl-4-hydroxybenzylmalonat, das Kondensationsprodukt aus 1-(2-Hydroxyethyl)-2,2,6,6-tetramethyl-4-hydroxypiperidin und Succinsäure, lineare oder zyklische Kondensationsprodukte von N,N'-Bis(2 ,2,6,6-tetramethyl-4-piperidyl )hexamethylendiamin und 4-*tert*-Octylamino-2,6-dichloro-1,3,5-triazin, Tris(2,2,6,6-tetramethyl-4-piperidyl)nitrilotriacetat, Tetrakis(2 ,2,6,6-tetramethyl-4-piperidyl)-1,2,3,4-butantetracarboxylat, 1,1'-(1,2-Ethandiyl)-bis(3,3,5,5-tetramethylpiper-azinon), 4-Benzoyl-2,2,6,6-tetramethylpiperidin, 4-Stearyloxy-2,2,6,6-tetramethylpiperidin, lineare oder zyklische Kondensationsprodukte aus N,N'-Bis(2,2,6,6-tetramethyl-4-piperidyl)hexamethylendiamin und 4-Morpholino-2,6-dichloro-1,3,5-triazin das Reaktionsprodukt von 7,7,9,9-Tetramethyl-2-cycloundecyl-1-oxa-3,8-diaza-4-oxospiro-[4,5]decan und Epichlorhydrin.

Bevorzugt sind oligomere und polymere gehinderte Amine der folgenden Strukturen:

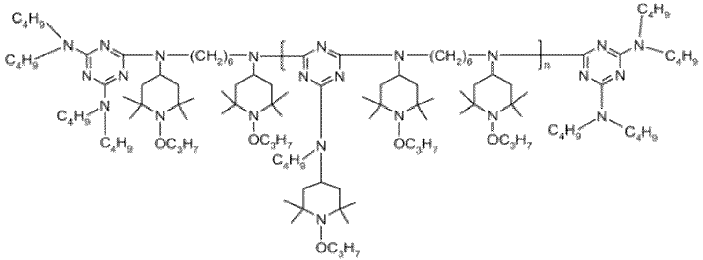

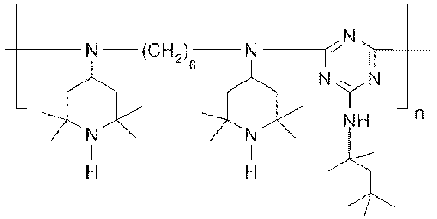

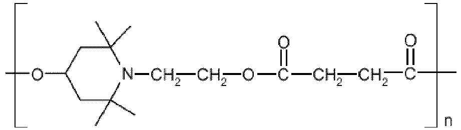

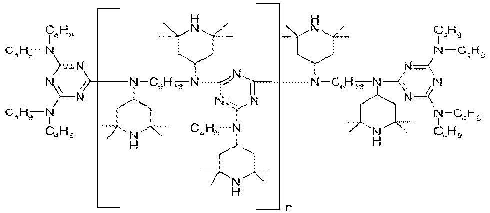

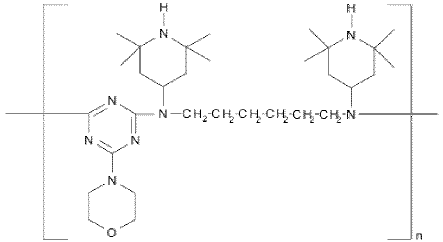

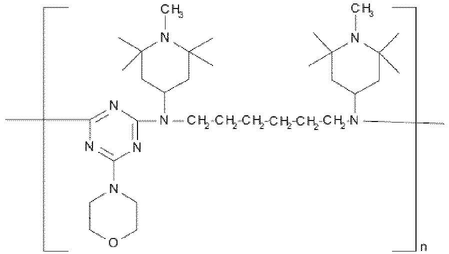

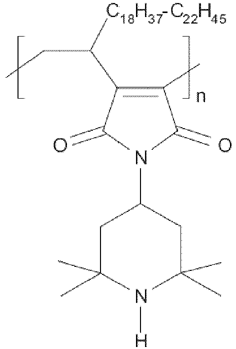

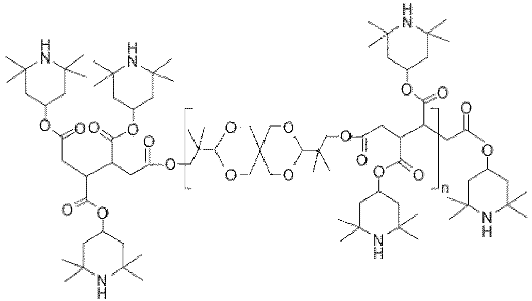

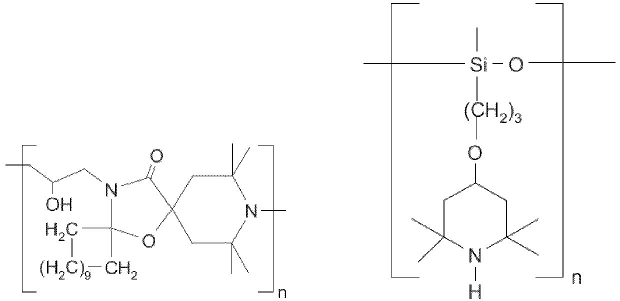

Bei den zuvor genannten Verbindungen bedeutet n jeweils 3 bis 100.

### Geeignete Dispergiermittel sind beispielsweise:

Polyacrylate, z.B. Copolymere mit langkettigen Seitengruppen, Polyacrylat-Blockcopolymere, Alkylamide: z.B. N,N'-1,2-Ethandiylbisoctadecanamid Sorbitanester, z.B. Monostearylsorbitanester, Titanate und Zirconate, reaktive Copolymere mit funktionellen Gruppen z.B. Polypropylen-co-Acrylsäure, Polypropylen-co-Maleinsäureanhydrid, Polyethylen-co-Glycidylmethacrylat, Polystyrol-alt-Maleinsäureanhydrid-Polysiloxane: z.B. Dimethylsilandiol-Ethylenoxid Copolymer, Polyphenylsiloxan Copolymer, Amphiphile Copolymere: z.B. Polyethylen-block-Polyethylenoxid, Dendrimere, z.B. hydroxylgruppenhaltige Dendrimere.

Geeignete Nukleierungsmittel sind beispielsweise Talkum, Alkali oder Erdalkalisalze von mono- und polyfunktionellen Carbonsäuren wie z.B. Benzoesäure, Bernsteinsäure, Adipinsäure, z.B. Natriumbenzoat, Zinkglycerolat, Alumi-niumhydroxy-bis(4-tert-butyl)benzoat, Benzylidensorbitole wie z.B. 1,3:2,4-Bis(Benzyliden)sorbitol oder 1,3:2,4-Bis(4-Methylbenzyliden)sorbitol, 2,2'-Methylen-bis-(4,6-di-*tert*-butylphenyl)phosphat, sowie Trisamide und Diamide wie z.B. Trimesinsäuretricyclohexylamid, Trimesinsäuretri(4-methylcyclohexylamid), Trimesinsäure tri(tert-butylamid), N,N',N"-1,3,5-Benzoltriyltris(2,2-dimethyl-propanamid) oder 2,6-Naphthalindicarbosäuredicyclohexylamid.

Geeignete Antinukleierungsmittel sind Azinfarbstoffe wie z.B. Nigrosin.

### Geeignete Flammschutzmittel sind beispielsweise:

a) Anorganische Flammschutzmittel wie z.B. Al(OH)₃, Mg(OH)₂, AlO(OH), MgCO₃, Schichtsilikate wie z.B. Montmorillonit oder Sepiolit, nicht oder organisch modifiziert, Doppelsalze, wie z.B. Mg-Al-Silikate, POSS-(Polyhedral Oligomeric Silsesquioxane) Verbindungen, Huntit, Hydromagnesit oder Halloysit sowie Sb₂O₃, SbzOs, MoO₃, Zinkstannat, Zinkhydroxystannat,
b) Stickstoffhaltige Flammschutzmittel wie z.B. Melamin, Melem, Melam, Melon, Melaminderivate, Melaminkondensationsprodukte oder Melaminsalze, Benzoguanamin, Polyisocyanurate, Allantoin, Phosphacene, insbesondere Melamincyanurat, Melaminphosphat, Dimelaminphosphat, Melaminpyrophosphat, Melaminpolyphosphat, Melamin-Metall-Phosphate wie z.B. Melaminaluminumphosphat, Melaminzinkphosphat, Melaminmagnesiumphopsphat, sowie die entsprechenden Pyrophosphate und Polyphosphate, Po-ly-[2,4-(piperazin-1,4-yl)-6-(morpholin-4-yl)-1,3,5-triazin], Ammoniumpolyphosphat, Melaminborat, Melaminhydrobromid,
c) Radikalbildner, wie z.B. Alkoxyamine, Hydroxylaminester, Azoverbindungen, Dicumyl oder Polycumyl, Hydroxyimide und deren Derivate wie z.B. Hydroxyimidester oder Hydroxyimidether
d) Phosphorhaltige Flammschutzmitteln wie z.B. roter Phosphor, Phosphate wie z.B. Resorcindiphosphat, Bisphenol-A-diphosphat und ihre Oligomere, Triphenylphosphat, Ethylendiamindiphosphat, Phosphinate wie z.B. Salze der hypophosphorigen Säure und Ihrer Derivate wie Alkylphosphinatsalzen z.B. Diethylphosphinataluminium oder Diethylphosphinat-Zink oder Aluminiumphosphinat, Aluminiumphosphit, Aluminiumphosphonat, Phosphonatester, oligomere und polymere Derivate der Methanphosphonsäure, 9,10-Dihydro-9-oxa-10-phosphorylphenanthren-10-oxid (DOPO) und deren substituierte Verbindungen,
e) Halogenhaltige Flammschutzmittel auf Chlor- und Brombasis wie z.B. polybrominierte Diphenyloxide, wie z.B. Decabromdiphenyloxid, Tris(3-bromo-2,2-bis(bromomethyl)propyl-phosphat, Tris(tribromneopentyl)phosphat, Tetrabromphthalsäure, 1,2-Bis-(tribromphenoxy)ethan, Hexabromcyclododecan, bromiertes Diphenylethan, Tris-(2,3-dibrompropyl)isocyanurat, Ethylen-bis-(tetrabromophthalimid), Tetrabromo-bisphenol A, bromiertes Polystyrol, bromiertes Polybutadien bzw, Polystyrol-bromiertes Polybutadien-Copolymere, bromierter Polyphenylenether, bromiertes Epoxidharz, Polypentabrombenzylacrylat, ggf. in Kombination mit Sb₂O₃ und/oder SbzOs,
f) Borate wie z.B. Zinkborat oder Calciumborat, ggf. auf Trägermaterial wie z.B. Silica
g) Schwefelhaltige Verbindungen wie z.B. elementarer Schwefel, Disulfide und Polysulfide, Thiuramsulfid, Dithiocarbamate, Mercaptobenzthiazol und Sulfenamide,
h) Antidrip-Mitteln wie z.B. Polytetrafluorethylen,
i) Siliciumhaltige Verbindungen wie z.B. Polyphenylsiloxane,
j) Kohlenstoffmodifikationen wie z.B. Carbon-Nanoröhren (CNT) oder Graphen
sowie Kombinationen oder Mischungen hieraus.

Geeignete Füllstoffe und Verstärkungsstoffe sind beispielsweise synthetische oder natürliche Materialien wie z.B. Calciumcarbonat, Silikate, Glasfasern, Glaskugeln (massiv oder hohl), Talkum, Glimmer, Kaolin, Bariumsulfat, Metalloxide und Metallhydroxide, Ruß, Graphit, Kohlenstoffnanoröhrchen, Graphen, Holzmehl oder Fasern von Naturprodukten wie z.B. Cellulose oder synthetische Fasern. Weitere geeignete Füllstoffe sind Hydrotalcite oder Zeolithe oder Schichtsilikate wie z.B. Montmorillonit, Bentonit, Beidelit, Mica, Hectorit, Saponit, Vermiculit, Ledikit, Magadit, Illit, Kaolinit, Wollastonit, Attapulgit.

Geeignete Pigmente können anorganischer oder organischer Natur sein. Anorganische Pigmente sind beispielsweise Titandioxid, Zinkoxid, Zinksulfid, Eisenoxid, Ultramarin, Ruß, organische Pigmente sind beispielsweise Anthrachinone, Anthanthrone, Benzimidazolone, Chinacridone, Diketopyrrolopyrrole, Dioxazine, Indanthrone, Isoindolinone, Azo-Verbindungen, Perylene, Phthalocyanine oder Pyranthrone. Weitere geeignete Pigmente sind Effektpigmente auf Metallbasis oder Perlglanzpigmente auf Metalloxid-Basis.

Geeignete Kettenverlängerer für den linearen Molekulargewichtsaufbau von Polykondensationspolymeren wir Polyestern oder Polyamiden sind beispielsweise Diepoxide, Bis-Oxazoline, Bis-Oxazolone, Bis-Oxazine, Diisocyanate, Dianhydride, Bis-Acyllactame, Bis-Maleimide, Dicyanate, Carbodiimide. Weitere geeignete Kettenverlängerer sind polymere Verbindungen wie z.B. Polystyrol-Polyacrylat-Polyglycidyl(meth)acrylat- Copolymere, Polystyrol-Maleinsäureanhydrid-Copolymere und Polyethylen-Maleinsäureanhydrid-Copolymere.

Geeignete Optische Aufheller sind beispielsweise Bisbenzoxazole, Phenylcumarine oder Bis(styryl)biphenyle und insbesondere optische Aufheller der Formeln:

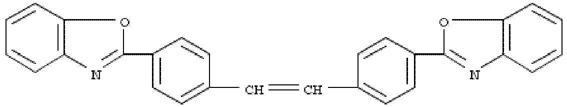

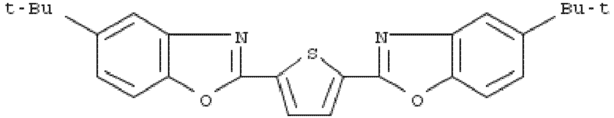

Geeignete Füllstoffdesaktivatoren sind beispielsweise Epoxide wie z.B. Bisphenol-A-diglycidylether, Polysiloxane, Polyacrylate insbesondere Blockcopolymere wie Polymethacrylsäure-polyalkylenoxid.

Geeignete Antistatika sind beispielsweise ethoxylierte Alkylamine, Fettsäureester, Alkylsulfonate und Polymere wie z.B. Polyetheramide.

Geeignete Antiozonantien sind die oben genannten Amine wie z.B. N,N'-Diisopropyl-p-phenylendiamin, N,N'-Di-sec-butyl-p-phenylendiamin, N,N'-Bis(1,4-dimethylpentyl)-p-phenylendiamin, N,N'-Dicyclohexyl-p-phenylendiamin, N-Isopropyl-N'-phenyl-p-phenylendiamin, N-(1,3-Dimethylbutyl)-N'-phenyl-p-phenylen-diamin, N-(1-Methylheptyl)-N'-phenyl-p-phenylendiamin, N-Cyclohexyl-N'-phenyl-p-phenylendiamin

Geeignete Entformungshilfsmittel sind beispielsweise Montanwachse.

Ganz besonders bevorzugt sind die nachfolgenden Zusatzstoffe, ausgewählt aus der Gruppe bestehend aus Phosphiten, Phosphoniten, Hydroxylaminen oder Nitronen in der erfindungsgemäßen Zusammensetzung enthalten.

Zudem betrifft die vorliegende Erfindung ein Verfahren zur Stabilisierung einer zu stabilisierenden organischen Komponente, insbesondere gegen oxidativen, thermischen oder actinischen Abbau oder Schädigung, bei dem mindestens eine erfindungsgemäße Verbindung mit der zu stabilisierenden organischen Komponente vermischt oder in diese eingearbeitet wird.

Bevorzugte Ausführungsformen eines derartigen Stabilisierungsverfahrens sehen beispielsweise vor, dass die erfindungsgemäße Verbindung als Pulver, Granulat, Lösung, Flüssigkeit, Suspension, Emulsion oder Schuppen vorliegen kann und mit der zu stabilisierenden organischen Komponente, insbesondere dem zu stabilisierenden Polymer oder Kunststoffgemisch, die Polymermatrix in die Schmelze überführt und anschließend abgekühlt wird. Alternativ hierzu ist es ebenso möglich, die Verbindung gemäß Formel I in einem schmelzflüssigen Zustand in eine Polymerschmelze einzubringen.

Für den Fall, dass der Polymerzusammensetzung weitere Bestandteile zugefügt werden, können diese separat, in Form von Flüssigkeiten, Pulvern, Dispersionen, Emulsionen, Granulaten oder kompaktierten Produkten oder zusammen mit der erfindungsgemäßen Additivzusammensetzung wie zuvor beschrieben den Polymeren zugesetzt werden.

Die Einarbeitung der oben beschriebenen erfindungsgemäßen Verbindungen und ggf. der zusätzlichen Additive in den Kunststoff erfolgt durch übliche Verarbeitungsmethoden, wobei das Polymer aufgeschmolzen und mit der erfindungsgemäßen Verbindung und den ggf. weiteren Zusätzen gemischt wird, vorzugsweise durch Mischer, Kneter und Extruder. Als Verarbeitungsmaschinen bevorzugt sind Extruder wie z.B. Einschneckenextruder, Zweischneckenextruder, Planetwalzenextruder, Ringextruder, Co-Kneter, die vorzugsweise mit einer Vakuumentgasung ausgestattet sind. Die Verarbeitung kann dabei unter Luft oder ggf. unter Inertgasbedingungen, z.B. unter Stickstoff erfolgen.

Weiterhin können die erfindungsgemäßen Verbindungen in Form von sogenannten Masterbatchen oder Konzentraten, die beispielsweise 10-90 % der erfindungsgemäßen Zusammensetzungen in einem Polymeren enthalten, hergestellt und eingebracht werden.

Zudem betrifft die vorliegende Erfindung die Verwendung der erfindungsgemäßen Verbindungen zur Stabilisierung organischer Materialien gegen oxidativen Abbau oder oxidative Schädigung.

Die vorliegende Erfindung wird anhand der nachfolgenden Ausführungen näher erläutert, ohne die Erfindung hierauf zu beschränken.

### Bevorzugte Ausführungen:

### Verfahren:

Bevorzugt erfolgt in einem ersten Reaktionsschritt die Umsetzung einer Allylverbindung oder Vinylverbindung oder Michael-Akzeptor mit einer Thiolverbindung, die zusätzlich eine Alkoholgruppe aufweist, mit Hilfe eines Katalysators und in einem zweiten Reaktionsschritt die Umesterung der Alkoholgruppe mit einer Esterverbindung, die eine sterisch gehinderte Phenolgruppe enthält. Besonders bevorzugt sind als Thiolverbindung 2-Mercaptoethanol, 3-Mercaptopropanol, 2-Mercaptopropanol und 1-Mercaptobutanol und Thioglycerin. Ein weiteres bevorzugtes Verfahren ist die Umsetzung einer Thiopropylverbindung, die eine sterisch gehinderte Phenolgruppe enthält, wie z.B.

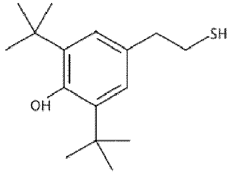

Bevorzugt wird die Reaktion in Anwesenheit eines Katalysators durchgeführt, bevorzugte Katalysatoren sind radikalerzeugende Verbindungen wie z.B. Photoinitiatoren, Azostarter oder Peroxide sowie für Michael-Akzeptoren Basen wie z.B. Natriummethanolat, Triethylamin oder Nucleophile wie z.B. 1-Methylimidazole oder Alkylphosphine wie z.B. Dimethylphenylphosphine.

Die Reaktion kann sowohl in Substanz als auch in einem Lösungsmittel durchgeführt werden, bevorzugte Lösungsmittel sind inerte Lösungsmittel wie z.B. Toluol, N-methyl-2-pyrrolidon oder N,N-dimethylformamid. Weiterhin findet Methanol als Lösungsmittel Verwendung. Besonders bevorzugte Reaktionen in Substanz werden vorzugsweise im Überschuss der Thiolkomponente als Lösungsmittel/Verdünner durchgeführt.

Der, wenn notwendig zweite Reaktionsschritt erfolgt vorzugsweise durch Umesterung der intermediär gebildeten Alkoholgruppe mit einem Ester, der die sterisch gehinderte Phenolgruppe trägt. Entsprechende Verfahren und Katalysatoren zu Umesterungsreaktionen sind dem Fachmann bekannt und beispielsweise in J. Otera, Chem. Rev. 1993, 93, 1449-1470 (s. Anlage) oder auch in WO 86/00301 (s. Anlage) oder WO2004/033699 (s. Anlage) beschrieben. Bevorzugte Katalysatoren sind Metallverbindungen wie z.B. aus der Gruppe der Zinnorganyle oder der Titanate.

### Verwendung:

Bevorzugte organische Materialien die mit den erfindungsgemäßen Stabilisatoren stabilisiert werden können, sind Kunststoffe, sowie Öle und Fette.

Handelt es sich bei den organischen Materialien um Öle und Fette, so können diese auf der Basis von Mineralölen, Pflanzenfetten oder Tierfetten sein oder auch Öle, Fette oder Wachse auf der Basis von z.B. synthetischen Estern. Pflanzliche Öle und Fette sind beispielsweise Palmöl, Olivenöl, Rapsöl, Leinöl, Sojabohnenöl, Sonnenblumenöl, Rizinusöl, Tierfette sind beispielsweise Fischöle oder Rindertalg.

Die erfindungsgemäßen Verbindungen können weiterhin als Stabilisatoren von Schmierstoffen, Hydraulikölen, Motorenölen, Turbinenölen, Getriebeölen Metallbearbeitungsflüssigkeiten oder als Schmierfette eingesetzt werden. Diese mineralischen oder synthetischen Schmierstoffe basieren vorwiegend auf Kohlenwasserstoffen.

### Ausführungsbeispiele

### I. Allgemeine Synthese-Vorschrift

Die erfindungsgemäßen Stabilisatoren werden dadurch hergestellt, dass zunächst eine Allyl/Vinyl-Verbindung in einem geeigneten Reaktionsgefäß mit einer Rühreinrichtung bei Raumtemperatur oder erhöhter Temperatur mit der Alkoholgruppe tragenden Thiolverbindung im leichten Überschuss vorzugsweise unter Zugabe eines Photokatalysators unter UV-Belichtung (Methode A), eines Radikalstarters unter Erwärmung (Methode B) oder für elektronenziehende Gruppen (EWG) vorzugsweise unter Zugabe einer Base (Methode C) nach folgendem Schema umgesetzt wird:

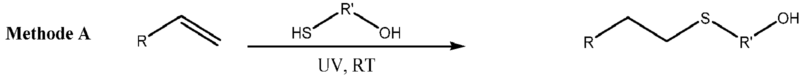

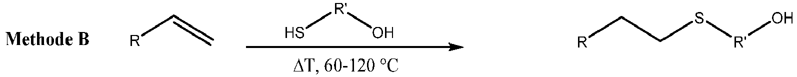

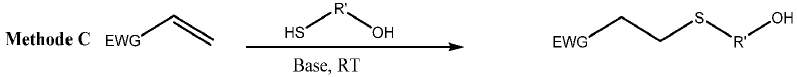

Vorzugsweise findet ein Entstabilisieren oder Entgasen der Edukte vor Reaktionsbeginn statt. Die Reaktions- und Belichtungsdauer der Methode A liegt bei 30-60 Minuten. Für Methode B beträgt die Reaktionszeit 4-35h. Zweckmäßig ist für Methode B eine Reaktion unter Inertgasbedingungen. Die Reaktionsdauer von Methode C beträgt wenige Minuten. Die Aufreinigung erfolgt wässrig oder über Hochvakuum bei 60-100 °C. Im zweiten Schritt erfolgt die Umesterung mit dem eine sterisch gehinderte Phenol Gruppe tragendem Ester im leichten Überschuss bei Temperaturen von 130-180°C unter Metallkatalyse nach dem Schema:

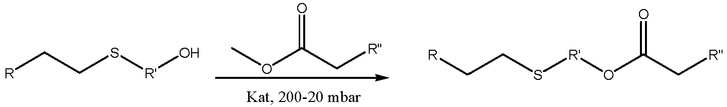

Der entstehende Alkohol wird durch Anlegen von Vakuum im Bereich 800-20 mbar aus dem Reaktionsgemisch entfernt. Die Aufreinigung erfolgt unter Hochvakuum oder wässrig. Eine Zugabe von Bleicherde oder eines anderen Adsorptionsmittels nach Aufnahme in Lösungsmittel ist zweckmäßig um metallische Katalysatorrückstände adsorptiv zu entfernen.

### II. Synthese erfindungsgemäßer Beispiele

### a) Synthese erfindungsgemäßes Beispiel A:

### Gemäß Schema 1

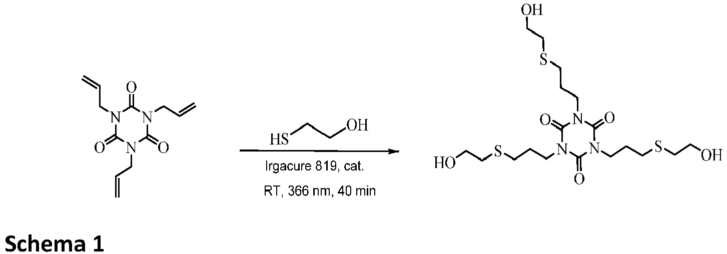

werden 15.0 g (0.06 mol, 1 Äq) Triallylisocyanurat mit 14.2 g (0.182 mol, 3.02 Äq) 2-Mercaptoethanol in einem Rundhalskolben mit Magnetrührer unter Rühren bei RT homogensiert. Der Mischung werden 0.1 wt.% Phenyl-Bis(2,4,6-trimethylbenzoyl)-phosphinoxid (Photoinitiator, bezogen auf das Isocyanurat) zugesetzt und es wird für 30-40 min unter UV-Belichtung bei λ = 366 nm und RT gerührt. Die Reaktionskontrolle erfolgt über ¹H NMR-Analyse. Die transparente, niederviskose Lösung wird in 150 ml Ethylacetat aufgenommen und zweimal mit je 50 ml dest. Wasser gewaschen, die organische Phase wird abgetrennt und das Lösungsmittel unter Hochvakuum bei 60 °C entfernt. Es werden 27.6 g (95 %) eines transparenten, hochviskosen Gels erhalten.

### Gemäß Schema 2

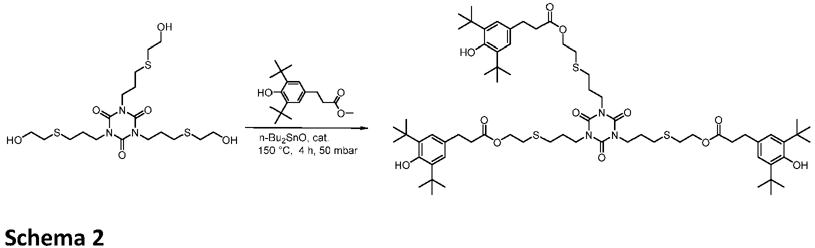

werden 10 g (0.021 mol, 1 Äq) des Thioethers mit 18.26 g (0.062 mol, 3. 02 Äq) 3-(3,5-Di-tert. Butyl-4-hydroxyphenyl)propionsäuremethylester in einem Schlenkkolben mit Magnetrührer, Kondensationsbrücke und Kühlfalle mit Vakuumanschluss bei 80 °C unter Stickstoff aufgeschmolzen. Zu der klaren Schmelze werden 0.15 mol% n-Bu₂SnO (bezogen auf den Thioether) zugegeben, die Reaktionsmischung auf 150 °C hochgeheizt und für ^{~}4 h bei ^{~}50 mbar gerührt. Die Reaktionskontrolle erfolgt durch ¹H NMR Analyse. Nach erfolgter Umsetzung wird das Vakuum auf <5 mbar erhöht und restlicher 3-(3,5-Di-tert. Butyl-4-hydroxyphenyl)propionsäuremethylester abkondensiert. Das Vakuum wird mit N₂ abgebrochen und die Reaktionsmischung auf RT abgekühlt wobei die Schmelze zu einer harten, glasartigen Masse erstarrt. Das Umesterungsprodukt wird in Toluol aufgenommen und zweimal mit 50 ml konz. NaHCO₃-Lösung und anschließend zweimal mit dest. Wasser bis zur pH-Neutralität gewaschen. Die organische Phase wird über Na₂SO₄ getrocknet, über eine Glasfilternutsche filtriert und das Lösungsmittel unter Vakuum entfernt. Es verbleibt ein harter, glasartig erstarrter Feststoff von 19.6 g (75%). Die Struktur wurde durch ihr ¹H NMR Spektrum bestätigt.

**¹H NMR** (300 MHz, CDCl₃) δ = 6.91 (s, 6H, -C_{arom.}-**H**), 5.00 (s, 3H, -C_{arom.}-O**H**), 4.15 (t, 6H, -COOC**H₂**-), 3.92 (t, 6H, -N-C**H₂**-), 2.79 (t, 6H, -C_{arom.}-C**H₂**-), 2.65 (t, 6H, -S-C**H₂**-CH₂O-), 2.59 - 2.44 (m, 12H, -N-(CH₂)₂-C**H₂**-, O=C-C**H₂**-), 1.87 (p, 6H, -N-CH₂-C**H₂**-CH₂-), 1.35 (s, 54H, -C**H₃**).

**¹³C NMR** (76 MHz, CDCl₃) δ = 173.08, 152.31, 149.09, 136.08, 131.10, 124.91, 63.41, 42.32, 36.50, 34.45, 31.08, 30.47, 29.64, 27.61.

### Gemäß Schema 3

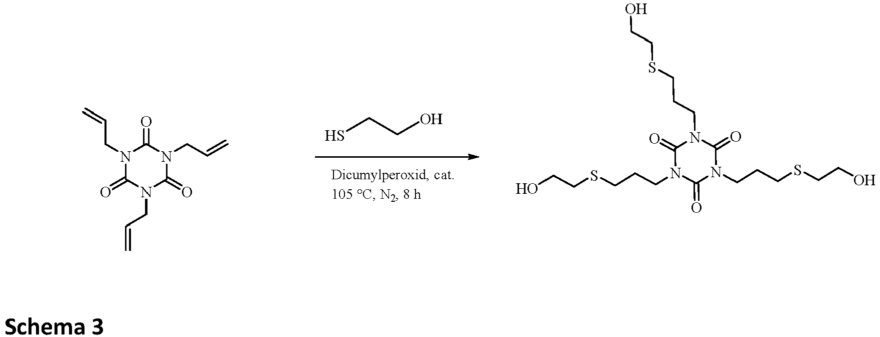

werden in einen 50 ml Schlenkkolben mit Septum 5.20 g (20.86 mmol, 1 Äq) Triallylisocyanurat mit 11.08 g (141.85 mmol, 6.8 Äq) 2-Mercaptoethanol unter Rühren gelöst und homogenisiert. Die klare Lösung wird für 1 h mit N2 entgast. Unter N2 wird das Septum gegen einen Rückflusskühler mit Blasenzähler ausgetauscht und die Lösung auf 105 °C hochgeheizt. Nach Erreichen der Temperatur werden 16 mg Dicumylperoxid zugegeben und die Reaktionsmischung für 8 h bei 105 °C gerührt. Die Reaktionskontrolle erfolgt über 1H NMR Analyse durch Abnahme der C=C Doppelbindungen. Die klare Lösung wird bei 80 °C unter Vakuum (10⁻² mbar) von überschüssigem Thiol befreit und aufgereinigt. Es verbleibt ein transparentes Gel von 9.97 g (97 % d.Th.).

### Gemäß Schema 4

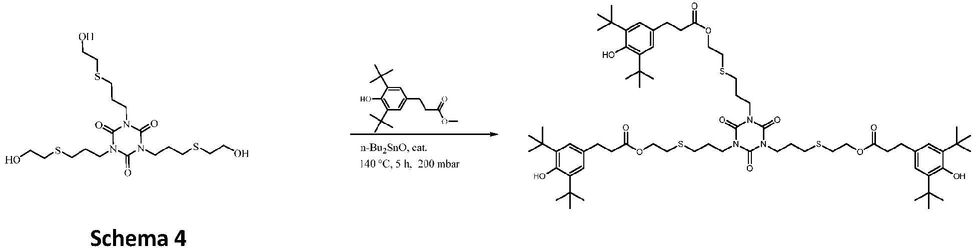

werden 7.65 g (15.82 mmol, 1 Äq) des Thioether-Precoursers mit 15.51 g (53.04 mmol, 3.35 Äq) 3-(3,5-Di-tert. Butyl-4-hydroxyphenyl)propion-säuremethylester (Metilox) in einem 100 ml Schlenkkolben mit Kondensationsbrücke und Kühlfalle mit Vakuumanschluss unter N₂ bei 80 °C aufgeschmolzen und homogenisiert. Zu der klaren Schmelze werden unter N₂ 156.83 mg (4.93 mmol, 0.31 Äq) DBTO zugegeben und die Temperatur auf 140 °C erhöht. Es wird ein leichtes Vakuum von ^{~}200 mbar angelegt und die Reaktionsmischung für 5 h gerührt. Nach Ende der Reaktionsdauer wird der Druck auf 10⁻³ mbar reduziert, die Temperatur auf 160 °C erhöht und überschüssiges Metilox abkondensiert. Nach Begasung mit N₂ wird die transparente Reaktionsschmelze auf RT abgekühlt und anschließend in Dichlormethan aufgenommen. Unter Rühren werden 1.4 g säureaktivierte Bleicherde (Optimum 210 FF, Clariant) zugegeben und die Supension für 30 min refluxiert. Es wird über ein kurzes Silica-Pad filtriert und das Lösemittel unter Vakuum entfernt. Es verbleibt ein glasartiger, transparenter Feststoff von 16.86 g (83 % d.Th.). Die Struktur wurde durch ihr 1H NMR Spektrum bestätigt.

**¹H NMR** (300 MHz, Chloroform-d) δ = 6.91 (s, 6H, -Carom.-H), 5.00 (s, 3H, - Carom.-OH), 4.15 (t, 6H, -COOCH2-), 3.92 (t, 6H, -N-C**H₂**-), 2.79 (t, 6H, -C_{arom.}-C**H₂**-), 2.65 (t, 6H, -S-C**H₂**-CH₂O-), 2.60 - 2.37 (m, 12H, -N-(CH₂)₂-C**H₂**-, O=C-C**H₂**-), 1.87 (p, 6H, -N-CH₂-C**H₂**-CH₂-), 1.35 (s, 54H, -C**H₃**).

**¹³C** NMR (76 MHz, Chloroform-d) δ = 172.98, 152.20, 148.98, 135.97, 130.99, 124.80, 63.29, 42.20, 36.38, 34.33, 30.95, 30.34, 29.52, 27.49 ppm.

### b) Synthese erfindungsgemäßes Beispiel B:

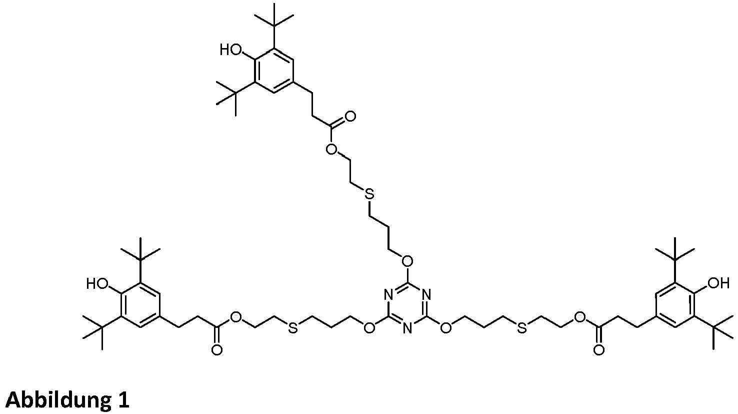

Analog Beispiel A Schema 1 werden 5.00 g (0.020 mol, 1 Äq) Triallylcyanurat mit 4.78 g (0.061 mol, 3.05 Äq) 2-Mercaptoethanol bei einer Belichtungsdauer von 50 min. umgesetzt. Die Aufreinigung erfolgt unter Hochvakuumbei 60 °C. Es werden 9.1 g (94 % d. Th.) eines transparenten, blassgelben und mittelviskosen Gels erhalten.

Analog Beispiel A Schema 2 werden 4 g (0.080 mol, 1 Äq) des Thioethers mit 7.74 g (0.026 mol, 3.25 Äq) 3-(3,5-Di-tert. Butyl-4-hydroxyphenyl)propion-säuremethylester bei ^{~}100 mbar umgesetzt und wässrig aufgearbeitet. Es verbleibt ein harter, gelb-transparenter Feststoff. Die Ausbeute beträgt 9.20 g (88 % d.Th.). Die Struktur nach Abbildung 1 wurde durch ihr ¹H NMR Spektrum bestätigt.

**¹H NMR** (300 MHz, CDCl₃) δ = 6.91 (s, 6H, -C_{arom.}-**H**), 5.00 (s, 3H, -Cₐᵣₒₘ.-O**H**), 4.14 (t, 6H, -COOC**H₂**-), 3.91 (t, 6H, -OC**H₂**-), 2.79 (t, 6H, -Cₐᵣₒₘ.-C**H₂**-), 2.65 (t, 6H, -S-C**H₂**-CH₂O-), 2.58 - 2.47 (m, 12H, -S-C**H₂**-(CH₂)₂O-, O=C-C**H₂**-), 1.87 (p, 6H, -O-CH₂-C**H₂**-CH₂-), 1.35 (s, 54H, -C**H₃**).

**¹³C NMR** (76 MHz, CDCl₃) δ = 172.97, 152.20, 135.97, 130.99, 124.80, 63.30, 42.21, 36.39, 34.34, 30.97, 30.36, 29.52, 27.50.

Analog Beispiel A Schema 3 werden 5.21 g (20.90 mmol, 1 Äq) Triallylcyanurat mit 7.97 g (102.01 mmol, 4.88 Äq) 2-Mercaptoethanol bei einer Reaktionstemperatur von 110 °C innerhalb einer Reaktionszeit von 31 h umgesetzt. Die Aufreinigung erfolgt unter Hochvakuum bei 100 °C. Es verbleibt ein transparentes Gel von 9.01 g (89 % d.Th.).

Analog Beispiel A Schema 4 werden 9.00 g (18.61 mmol, 1 Äq) des Thioethers mit 22.40 g (76.60 mmol, 4.12 Äq) 3-(3,5-Di-tert. Butyl-4-hydroxyphenyl)propionsäuremethylester bei 50-200 mbar umgesetzt und wie beschrieben mit 2.8 g Bleicherde aufgereinigt. Die Ausbeute des glasartigen, transparenten und hochviskosen Harzes beträgt 14.27 g (61 % d. Th.). Die Struktur nach Abbildung 1 wurde durch ihr ¹H NMR Spektrum bestätigt.

**¹H** NMR (300 MHz, Chloroform-d) δ = 6.91 (s, 6H, -Cₐᵣₒₘ.-**H**), 5.00 (s, 3H, -C_{arom.}-**OH),** 4.40 (t, 6H, -C_{arom.}-O-C**H₂**- ), 4.16 (t, 6H, -COOC**H₂**-), 2.79 (t, 6H-C_{arom.}-C**H₂**-), 2.65 (td, 12H, -C**H₂**-S-C**H₂**), 2.57 - 2.50 (m, 6H, O=C-C**H₂**-), 1.99 (p, 6H, -O-CH₂-C**H₂**-CH₂-), 1.35 (s, 54H, -C**H₃**) ppm.

**¹³C** NMR (76 MHz, Chloroform-d) δ = 173.01, 172.98, 152.20, 135.97, 130.98, 124.80, 66.73, 63.33, 36.37, 34.33, 30.96, 30.50, 30.34, 28.64 ppm.

### c) Synthese erfindungsgemäßes Beispiel C:

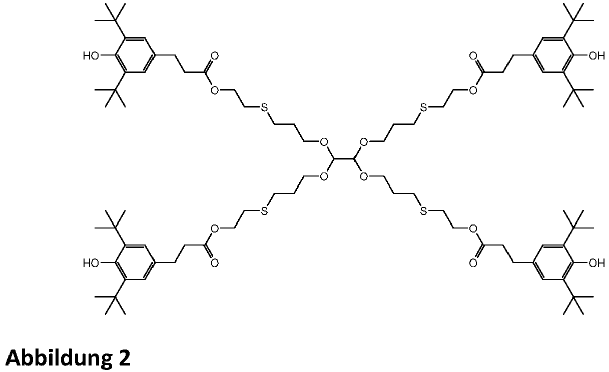

Analog Beispiel A Schema 1 werden 7.0 g (0.028 mol, 1 Äq) Tetraallyloxyethan mit 8.8 g (0.113 mol, 4.1 Äq) 2-Mercaptoethanol umgesetzt. Die Belichtungsdauer beträgt 60 min. Nach wässriger Aufarbeitung analog Beispiel A wird unter Hochvakuum bei 80 °C weiter aufgereinigt und vom Lösungsmittel befreit. Es werden 13.6 g (87 % d. Th.) eines transparenten, bernsteinfarbenen und niederviskosen Gels erhalten.

Analog Beispiel A Schema 2 werden 3.52 g (0.006 mol, 1 Äq) des Thioethers mit 7.72 g (0.025 mol, 4.0 Äq) 3-(3,5-Di-tert.-butyl-4-hydroxyphenyl)propion-säuremethylester bei 140 °C und ^{~}80 mbar umgesetzt. Das Rohprodukt wird in ca. 200 ml Ethylacetat gelöst und analog Beispiel A wässrig aufgearbeitet. Es werden 9.25 g (92 % d. Th.) eines transparenten, bernsteinfarbenen und hochviskosen Gels erhalten. Die Struktur nach Abbildung 2 wurde durch ihr ¹H NMR Spektrum bestätigt.

**¹H NMR** (300 MHz, CDCl₃) δ = 6.91 (s, 8H, -Cₐᵣₒₘ.-**H**), 5.00 (s, 4H, -Cₐᵣₒₘ.-O**H**), 4.25 (s, 2H,-C**H**-C**H**-), 4.15 (t, 8H, -COOC**H₂**-), 3.76 - 3.46 (m, 8H, -CH-O-C**H**₂), 2.79 (t, 8H, -C_{arom.}-C**H₂**-), 2.64 (t, 8H, O=C-C**H**₂-)2.70 - 2.42 (m, 16H, -C**H₂**-S-C**H₂**-), 1.79 (p, 8H, -S-CH₂-C**H₂**-CH₂-O-), 1.35 (s, 72H, -C**H₃**).

**¹³C NMR** (76 MHz, CDCl₃) δ = 172.97, 152.19, 135.94, 130.99, 124.79, 102.37, 66.01, 63.36, 36.38, 34.33, 30.96, 30.34, 29.84, 28.92.

Analog Beispiel A Schema 3 werden 5.00 g (19.66 mmol, 1 Äq)Tetraallyloxyethan mit 7.37 g (94.33 mmol, 4.8 Äq) 2-Mercaptoethanol und 216 mg (0.80 mmol, 0.04 Äq) Dicumylperoxid bei einer Reaktionstemperatur von 100 °C für 2 h gerührt. Es werden erneut 1.34 g (17.15 mmol, 1.15 Äq) 2-Mercaptoethanol zugesetzt und für weitere 2.5 h gerührt. Die Aufreinigung erfolgt unter Hochvakuum bei 100 °C. Es verbleibt ein transparentes, leicht gelbes Gel von 10.07 g (90 % d.Th.).

Analog Beispiel A Schema 4 werden 8.59 g (15.15 mmol, 1 Äq) des Thioethers mit 19.50 g (66.68 mmol, 4.4 Äq) 3-(3,5-Di-tert. Butyl-4-hydroxyphenyl)-propionsäuremethylester bei 50-200 mbar umgesetzt und wie beschrieben mit 2.8 g Bleicherde aufgereinigt. Die Ausbeute des glasartigen, transparenten und leicht gelben, hochviskosen Harzes beträgt 14.27 g (61 % d. Th.). Die Struktur nach Abbildung 2 wurde durch ihr ¹H NMR Spektrum bestätigt.

**¹H** NMR (300 MHz, Chloroform-d) δ = 6.91 (s, 8H, -Cₐᵣₒₘ.-**H**), 5.00 (s, 4H, -C_{arom.}-O**H**), 4.25 (s, 2H,-C**H**-C**H**-), 4.15 (t, 8H, -COOC**H₂**-), 3.75 - 3.43 (m, 8H, -CH-O-C**H₂**), 2.80 (t, 8H, -Cₐᵣₒₘ.-C**H₂**-), 2.64 (t, 8H, O=C-C**H₂**-), 2.61 - 2.48 (m, 16H, - C**H₂**-S-C**H₂** -), 1.79 (p, 8H, -S-CH₂-C**H₂**-CH₂-O-), 1.35 (s, 72H, -C**H₃**) ppm.

**¹³C** NMR (76 MHz, Chloroform-d) δ = 172.97, 152.20, 135.95, 131.00, 124.80, 102.38, 66.02, 63.36, 36.38, 34.33, 30.96, 30.35, 29.85, 28.92.

### d) Synthese nicht-erfindungsgemäßes Beispiel D:

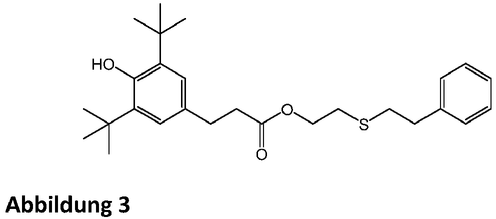

Analog Beispiel A Schema 1 werden 5.71 g (0.055 mol, 1 Äq) Styrol mit 4.71 g (0.060 mol, 1.1 Äq) 2-Mercaptoethanol und 0.2 wt.% Phenyl-Bis(2,4,6-trimethylbenzoyl)-phosphinoxid (Photoinitiator, bezogen auf das Styrol) umgesetzt. Das transparente, niederviskose Rohprodukt wird in ca. 100 ml Dichlormethan aufgenommen und analog Beispiel A wässrig aufgearbeitet. Es werden 7.48 g (75 % d. Th.) eines transparenten niederviskosen Gels erhalten.

Analog Beispiel A Schema 2 werden 4.12 g (0.023 mol, 1 Äq) des Thioethers mit 7.72 g (0.025 mol, 1.1 Äq) 3-(3,5-Di-tert. Butyl-4-hydroxyphenyl)propionsäuremethylester bei 140 °C und ^{~}800 mbar umgesetzt. Das Rohprodukt wird in ca. 150 ml Toluol aufgenommen und analog Beispiel A wässrig aufgearbeitet. Es werden 9.27 g (93 % d. Th.) eines transparenten, gelblichen und hochviskosen Gels erhalten. Die Struktur nach Abbildung 3 wurde durch ihr ¹H NMR Spektrum bestätigt.

**¹H NMR** (300 MHz, CDCl₃) δ = 7.29 - 7.05 (m, 5H, -C_{arom., Styrol}-**H**), 6.91 (s, 2H, - C_{arom., Metilox}-**H**), 4.99 (s, 1H, -C_{arom., Metilox}-O**H**), 4.15 (t, 2H, COOC**H₂**-), 2.86 - 2.68 (m, 6H, -C**H₂**-S-C**H₂**-C**H₂**-), 2.65 (t, 2H, C_{arom., Metilox}-C**H₂**-), 2.59 - 2.46 (m, 2H, O=C-C**H₂**-), 1.35 (s, 18H, -C**H₃**).

**¹³C NMR** (76 MHz, CDCl₃) δ = 140.23 , 128.63 , 128.54, 128.50, 128.35 , 126.49, 125.85 , 60.30, 36.37, 35.50, 33.22.

### e) Synthese erfindungsgemäßes Beispiel E:

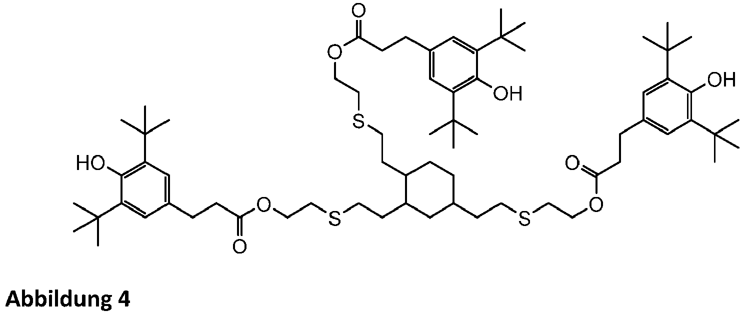

Analog Beispiel A Schema 1 werden 6.00 g (0.037 mol, 1 Äq) Trivinylcyclohexan mit 8.81 g (0.113 mol, 3.05 Äq) 2-Mercaptoethanol mit 0.2 wt% Phenyl-Bis(2,4,6-trimethylbenzoyl)-phosphinoxid (Photoinitiator, bezogen auf das Trivinylcyclohexan) umgesetzt. Das transparente, mittelviskose und blassgelbe Rohprodukt wird in ca. 100 ml Diethylether aufgenommen und analog Beispiel A wässrig aufgearbeitet. Nach weiterer Aufreinigung unter Hochvakuum bei 80 °C, werden 13.8 g (94 % d. Th.) eines transparenten, blassgelben und mittelviskosen Gels erhalten.

Analog Beispiel A Schema 2 werden 4.00 g (0.010 mol, 1 Äq) des Thioethers mit 8.99 g (0.031 mol, 3.05 Äq) 3-(3,5-Di-tert. Butyl-4-hydroxyphenyl)-propionsäuremethylester bei 150 °C und ^{~} 100 mbar umgesetzt. Die Aufreinigung erfolgt analog Beispiel A wässrig. Es verbleibt ein zähes, gelbtransparentes Harz mit einer Ausbeute von 10.21 g (86 % d.Th.). Die Struktur nach Abbildung 4 wurde durch ihr ¹H NMR Spektrum bestätigt.

**¹H NMR** (300 MHz, CDCl₃) δ = 6.91 (s, 6H, C_{arom.}-**H**), 5.00 (s, 3H, -Cₐᵣₒₘ.-O**H**), 4.15 (t, 6H, -COOC**H₂**-), 2.90 - 2.73 (t, 6H, -C_{arom.}-C**H₂**), 2.64 (t, 6H, O=C-C**H**₂-), 2.60 - 2.31 (m, 12H, -C**H₂**-S-C**H₂**-), 1.35 (s, 54H, -C**H₃**), 1.99 - 0.38 (m, 15H, - C**H₂**-C**H**_{Ring, aliph}., C**H**_{**2**,Ring, aliph}.).

**¹³C NMR** (76 MHz, CDCl₃) δ = 173.00, 152.20, 135.97, 131.01, 124.81, 63.45, 40.71, 38.44, 37.07, 36.61, 36.42, 34.35, 33.28, 32.39, 31.00, 30.61, 30.38, 29.91, 29.54.

### f) Synthese erfindungsgemäßes Beispiel F:

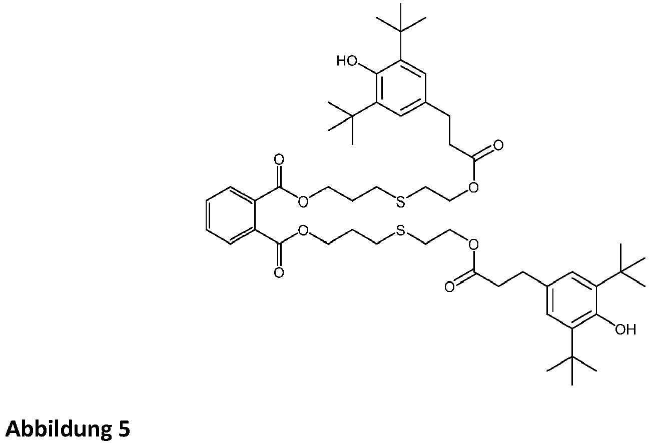

Analog Beispiel A Schema 1 werden 6.12 g (0.025 mol, 1 Äq) Diallylphthalat mit 4.08 g (0.052 mol, 2.1 Äq) 2-Mercaptoethanol mit 0.2 wt.% Phenyl-bis(2,4,6-trimethylbenzoyl)-phosphinoxid (Photoinitiator, bezogen auf das Diallylphthalat) umgesetzt. Die Aufreinigung erfolgt unter Hochvakuumbei 60 °C. Es werden 9.91 g (99 % d. Th.) eines transparenten, gelblichen und niederviskosen Gels erhalten.

Analog Beispiel A Schema 2 werden 3.50 g (0.009 mol, 1 Äq) des Thioethers mit 5.09 g (0.174 mol, 2.0 Äq) 3-(3,5-Di-tert. Butyl-4-hydroxyphenyl)propion-säuremethylester bei 140 °C und ^{~} 800 mbar umgesetzt. Die Aufreinigung erfolgt unter Hochvakuumbei 140 °C. Es werden 7.38 g (74 % d. Th.) eines transparenten, gelblichen und niederviskosen Gels erhalten. Die Struktur nach Abbildung 5 wurde durch ihr ¹H NMR Spektrum bestätigt.

**¹H NMR** (300 MHz, CDCl₃) δ = 7.64 (m, 2H,-C-C_{arom., Phthalat}-**H**), 7.55 - 7.37 (m, 2H, -C_{arom., Phthalat}-**H**), 6.91 (s, 4H, -C_{arom., Metilox}-**H**), 5.00 (s, 2H, -Cₐᵣₒₘ.-O**H**), 4.44 - 4.28 (m, 4H, -C_{arom., Phthalat-}COOC**H₂**-), 4.22 - 4.04 (m, 4H, -CH₂COOC**H₂**-), 2.79 (t, 6H, -Cₐᵣₒₘ.-C**H₂**-), 2.70 - 2.48 (m, 12H), 1.96 (p, 4H, -S-CH₂-C**H₂**-CH₂-), 1.35 (s, 36H, -C**H₃**).

**¹³C NMR** (76 MHz, CDCl₃) δ = 172.97 , 167.40 , 152.19 , 135.95 , 131.99 , 131.14, 130.97 , 128.91 , 124.79, 64.12, 63.33 , 62.83 , 36.37 , 34.32 , 30.95 , 30.48, 30.34, 28.74, 28.67.

Analog Beispiel A Schema 3 werden 9.18 g (37.28 mmol, 1 Äq) Diallylphthalat mit 6.99 g (89.47 mmol, 2.4 Äq) 2-Mercaptoethanol und 180 mg (1.1 mmol, 0.02 Äq) 2,2'-Azobis(2-methylpropionitril) bei einer Reaktionstemperatur von 70 °C für 1 h gerührt. Es werden erneut 0.04 g (0.24 mmol, 0.006 Äq) 2,2'-Azobis(2-methylpropionitril) zugesetzt und für weitere 9 h bei 80 °C gerührt. Die Aufreinigung erfolgt unter Hochvakuum bei 80 °C. Es verbleibt ein transparentes, leicht gelbes niederviskoses Gel von 13.68 g (91 % d.Th.).

Analog Beispiel A Schema 4 werden 8.72 g (21.66 mmol, 1 Äq) des Thioethers mit 12.99 g (44.42 mmol, 2.05 Äq) 3-(3,5-Di-tert. Butyl-4-hydroxyphenyl)-propionsäuremethylester bei 50-200 mbar umgesetzt und wie beschrieben mit 1.4 g Bleicherde aufgereinigt. Die Ausbeute des transparenten und leicht gelben, hochviskosen Harzes beträgt 8.77 g (87 % d. Th.). Die Struktur nach Abbildung 5 wurde durch ihr ¹H NMR Spektrum bestätigt.

**¹H** NMR (300 MHz, Chloroform-d) δ = 7.72 - 7.60 (m, 2H, -C-C_{arom., Phthalat}-**H**), 7.53 - 7.39 (m, 2H, -C_{arom., Phthalat}-**H**), 6.92 (s, 4H, -C_{arom., Metilox}-**H**), 5.00 (s, 2H, - C_{arom.}-O**H**), 4.35 (t, 4H, -C_{arom., Phthalat-}COOC**H₂**-), 3.66 (t, 4H, -CH₂COOC**H₂**-), 2.80 (t, 4H, C_{arom.}-C**H₂**-), 2.64 - 2.49 (m, 8H, -C**H₂**-S-C**H₂**), 1.97 (p, 4H, -S-CH₂-C**H₂**-CH₂-), 1.36 (s, 36H, -C**H₃**).

### g) Synthese nicht-erfindungsgemäßes Beispiel G:

### Gemäß Schema 5

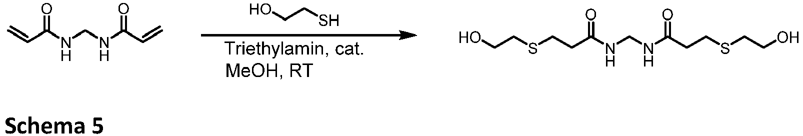

werden 5.40 g (35.03 mmol, 1 Äq) N,N'-Methylenbisacrylamid mit 12.21 g (156.28 mmol, 4.5 Äq) 2-Mercaptoethanol und 370 mg (3.6 mmol, 0.1 Äq) Triethylamin in 20 ml Methanol gerührt und suspendiert. Die Supension klärt unter Erwärmung homogen und transparent auf, wobei nach wenigen Minuten ein weißer Feststoff ausfällt. Der Feststoff wird abfiltriert, dreimal mit 15 ml destilliertem Wasser und 15 ml Methanol gewaschen und im Trockenschrank getrocknet. Es verbleibt ein weißer Feststoff von 7.40g (68 % d.Th.).

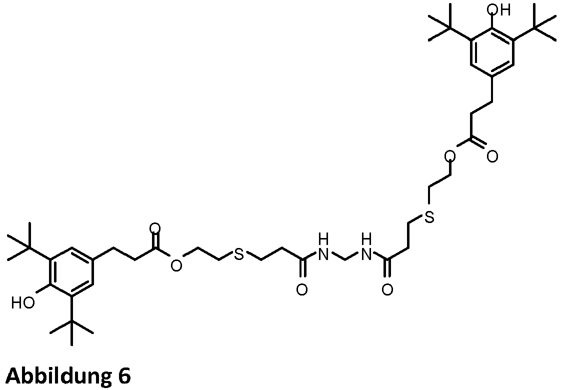

Analog Beispiel A Schema 4 werden 6.95 g (22.38 mol, 1 Äq) des Thioethers mit 31.30 g (107.04 mol, 4.8 Äq) 3-(3,5-Di-tert. Butyl-4-hydroxyphenyl)-propionsäuremethylester bei 160 °C und 50-200 mbar innerhalb von 8 h umgesetzt. Die Aufreinigung erfolgt wie beschrieben mit 1.4 g Bleicherde. Es werden 16.72 g (90 % d. Th.) eines glasartigen, hellbraunen Feststoffs erhalten. Die Struktur nach Abbildung 6 wurde durch ihr ¹H NMR Spektrum bestätigt.

**¹H** NMR (300 MHz, Chloroform-*d*) δ = 6.91 (s, 4H, -Cₐᵣₒₘ.-**H**), 6.87 (t, 2H, -N**H-**CH₂-N**H**-), 5.02 (s, 2H, -Cₐᵣₒₘ.-O**H**), 4.54 (t, 2H, -NH-C**H₂**-NH-), 4.15 (t, 4H, -C_{arom.-}COOC**H₂**-), 2.86 - 2.73 (m, 8H, -NH-CO-C**H₂**-, -C_{arom.}-CH₂-C**H₂**-), 2.54 (t, 4H, - C_{arom.}-C**H₂**) 2.39 (t, 4H, C_{arom.}-C**H₂**-), 1.35 (s, 36H, -C**H₃**).

**¹³C** NMR (76 MHz, Chloroform-*d*) δ = 173.06, 172.06, 152.21, 136.01, 130.96, 124.80, 63.37, 44.67, 36.42, 36.37, 34.33, 30.94, 30.74, 30.35, 27.67 ppm.

### h) Synthese nicht-erfindungsgemäßes Beispiel H:

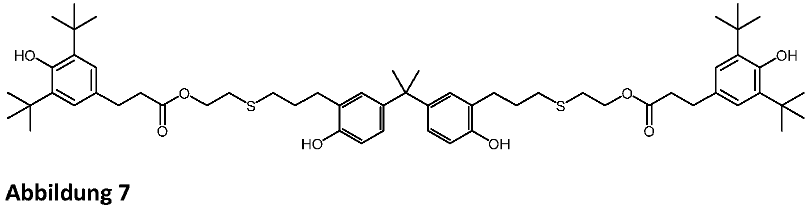

Analog Beispiel A Schema 1 werden 6.64 g (21.53 mol, 1 Äq) o,o'-Diallylbisphenol A mit 3.45 g (44.16 mol, 2.05 Äq) 2-Mercaptoethanol mit 0.1 wt.% Phenyl-Bis(2,4,6-trimethylbenzoyl)-phosphinoxid (Photoinitiator, bezogen auf das Diallyl) umgesetzt. Die Aufreinigung erfolgt unter Hochvakuum bei 60 °C. Es werden 9.04 g (90 % d. Th.) eines transparenten, gelblichen und niederviskosen Gels erhalten.

Analog Beispiel A Schema 2 werden 3.25 g (6.99 mol, 1 Äq) des Thioethers mit 4.29 g (14.69 mol, 2.1 Äq) 3-(3,5-Di-tert. Butyl-4-hydroxyphenyl)propionsäure-methylester bei 145 °C und 50-200 mbar innerhalb 5 h umgesetzt. Die Aufreinigung erfolgt unter Hochvakuumbei 150 °C. Es werden 6.07 g (88 % d. Th.) eines transparenten, gelblichen und hochviskosen Harzes erhalten. Die Struktur nach Abbildung 7 wurde durch ihr 1H NMR Spektrum bestätigt.

**¹H** NMR (300 MHz, Chloroform-d) δ = 6.91 (s, 4H, -C_{arom.}-C**H**), 6.87 - 6.70 (m, 4H, -C**H₂**-C_{Bisphenol}-C**H₂** ), 6.57 (s, 2H, -C_{Bisphenol}OH-C**H₂**-), 5.15 (s, 2H, -C_{Bisphenol}-O**H**), 5.00 (s, 2H, -Cₐᵣₒₘ.-O**H**), 4.14 (t, 4H, -COO-C**H₂**-CH₂-S-), 2.80 (t, 4H, -C_{arom.}-C**H₂**-CH₂-COO-), 2.68 - 2.50 (m, 12H), 2.46 (t, 4H, -C_{arom.}-C**H₂**-CH₂-COO-), 1.80 (p, 4H, , -S-CH₂-C**H₂**-CH₂-), 1.52 (s, 6H, -C_{Bisphenol}**H₃**), 1.35 (s, 36H, -C**H₃**).

**¹³C** NMR (76 MHz, Chloroform-d) δ = 173.29, 152.22, 151.57, 143.31, 135.98, 130.97, 128.84, 126.45, 125.64, 114.97, 63.53, 41.66, 36.40, 34.33, 31.69, 30.95, 28.93.

### i) Synthese erfindungsgemäßes Beispiel I:

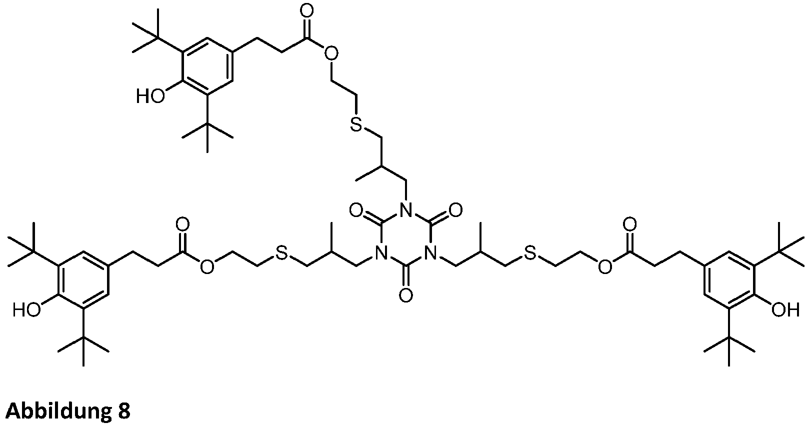

Analog Beispiel A Schema 3 werden 5.56 g (19.08 mmol, 1 Äq) Trimethylallylisocyanurat mit 9.63 g (123.26 mmol, 6.46 Äq) 2-Mercaptoethanol und 160 mg (0.60 mmol, 0.03 Äq) Dicumylperoxid bei einer Reaktionstemperatur von 115 °C für 8 h gerührt. Die Aufreinigung erfolgt unter Hochvakuum bei 80 °C. Es verbleibt ein transparentes, leicht gelbes Gel von 7.14 g (71 % d.Th.). Analog Beispiel A Schema 4 werden 7.00 g (13.31 mmol, 1 Äq) des Thioethers mit 16.24 g (55.54 mmol, 4.2 Äq) 3-(3,5-Di-tert. Butyl-4-hydroxyphenyl)-propionsäuremethylester bei 50-200 mbar umgesetzt und wie beschrieben mit 2.8 g Bleicherde aufgereinigt. Die Ausbeute des glasartigen, transparenten und leicht orangenen, glasartigen Feststoffs beträgt 16.29 g (94 % d.Th.). Die Struktur nach Abbildung 8 wurde durch ihr 1H NMR Spektrum bestätigt.

**¹H** NMR (300 MHz, Chloroform-*d*) δ = 6.91 (s, 6H, -C_{arom.}-C**H**), 5.00 (s, 3H, -C_{arom.}-O**H**), 4.12 (t, 6H, -COO-C**H₂**-CH₂-S-), 3.89 (dd, 3H, -N-C**H₂**'-), 3.73 (dd, 3H, -N-CH₂"-), 2.79 (t, 6H, -Cₐᵣₒₘ.-C**H₂**-), 2.63 (t, 6H, -COO-CH₂-C**H₂**-S-), 2.53 (m, 9H, -C_{arom.}-CH₂-C**H₂**-, -S-C**H₂**'-CH-), 2.41 (dd, 3H, -S-C**H₂**"-CH-), 2.18 (p, 3H, -N-CH₂- C**H**-), 1.35 (s, 54H, -C**H₃**), 0.95 (d, 9H, -CH-C**H₃**).

### j) Synthese erfindungsgemäßes polymeres Beispiel J:

In einem ausgeheizten 100 mL-Schlenkkolben werden 20.88 (83.76 mmol, 1 Äq) Triallylisocyanurat in 6.66 g (85.24 mmol, 0.98 Äq) 2-Mercaptoethanol gelöst. Die Reaktionsmischung wird 2-mal nach der *Freeze-Pump-Thaw-*Methode entgast. Nach der Zugabe einer Spatel-Spitze Phenyl-Bis(2,4,6-trimethylbenzoyl)-phosphinoxid (Photoinitiator) wird die Reaktionsmischung 1 h mit UV-Licht der Wellenlänge 366 nm bestrahlt. Dabei ist nach einer halben Stunde eine deutliche Zunahme der Viskosität der Reaktionsmischung zu beachten. Anschließend wird die Reaktionsmischung auf 110 °C aufgeheizt und es werden 0.1 mL *2,5-Dimethyl-2,5-di-(tert-butylperoxy)-hexan (Radikalstarter)* zugegeben. Nach 2 h Reaktionszeit werden nochmals 1 ml 2,5-*Dimethyl-2,5-di-(tert-butylperoxy)-hexan* zugegeben. Die viskose, leicht gelbe Reaktionsmischung wird nach einer Reaktionsdauer von 17 h in 250 ml kaltem Methanol gefällt. Nach dem Trocknen unter Hochvakuum werden 3.80 g eines farblosen, pulverförmigen Produktes erhalten.

In einem 100 mL-Schlenkkolben werden 2.76 g des polymeren Thioethers und 16.16 g 3-(3,5-Di-tert. Butyl-4-hydroxyphenyl)propionsäuremethylester in 40 ml Chloroform gelöst. Die Lösung wird auf 100 °C im Rückfluss erhitzt und es werden 100 mg Dibutylzinnoxid zugegeben. Nach 26 h wird die Reaktionsmischung auf Raumtemperatur abgekühlt und in 300 ml kaltes Methanol geschüttet. Das ausfallende Produkt wird abfiltriert, mit Methanol gewaschen und anschließend unter Hochvakuum getrocknet. Es werden 2.02 g des weißen, pulverförmigen Produktes erhalten. Die polymere Verbindung wurde über ihr FTIR-Spektrum bestätigt.

**IR:** 3521 v(-OH), 2953 v(-CH₂), 1670 v(-C_{Ester}=O), 1155 v (para subst. Aromat), 1450 v(-C-N), 931 v(=CH), 762 v(-C-S-), 697 v(=C-H mono-subst. Benzol) cm⁻¹.

### III. Anwendungsprüfung der erfindungsgemäßen Beispiele

### a) Anwendungsprüfung des erfindungsgemäßen Beispiels A

Das erfindungsgemäße Beispiel A wurde mit den in Tabelle 1 ersichtlichen Gewichtsprozenten in Polypropylen (Moplen HP 500N, Lyondell Basell Industries) im Vergleich zu einem handelsüblichen schwefelhaltigen phenolischen Antioxidans (Vergleichsbeispiel 2, Songnox 1035, Songwon) eingearbeitet. Das erfindungsgemäße Beispiel 1 zeigt die vergleichbare Gewichtskonzentration zum Vergleichsbeispiel, das erfindungsgemäße Beispiel 2 wurde nach dem Moläquivalent an phenolischen Antioxidansgruppen berechnet.

**Tabelle 1: Zusammensetzungen der erfindungsgemäßen Beispiele und Vergleichsbeispiele**

| | |
|---|---|
| Vergleichsbeispiel 1 | Ohne Zusatz |
| Vergleichsbeispiel 2 | 0.3 % Songnox 1035 |
| Vergleichsbeispiel 3 | 0.1% Irganox 1010 / 0.2 % DSTDP |
| Vergleichsbeispiel 4 | 0.1% Irganox 1076 / 0.2 % DLTDP |
| Erfindungsgemäßes Beispiel 1 | 0.3 % Beispiel A |
| Erfindungsgemäßes Beispiel 2 | 0.39 % Beispiel A |

Unter Songnox 1035 wird die folgende Struktur verstanden: 2,2'-Thiodiethylen bis[3-(3,5-di-tert-butyl-4-hydroxyphenyl)propionat]
DSTDP: Distearylthiodipropionat
DLDTP: Dilauryldithiopropionat

Die Extrusion der Compound-Mischungen erfolgte in einem Doppelschneckenextruder vom Typ Process 11 (Fisher Scientific) unter dem in Tabelle 2 aufgeführtem Temperaturprofil. Die Polymerschmelze wird mit 200 U/min ausgetragen und im Wasserbad abgekühlt sowie anschließend granuliert.

**Tabelle 2: Temperaturprofil der Extrusionsversuche**

| | |
|---|---|
| Zone 1 | 170 °C |
| Zone 2 | 180 °C |
| Zone 3 | 190 °C |
| Zone 4 | 200 °C |
| Zone 5 | 200 °C |
| Zone 6 | 200 °C |
| Zone 7 | 200 °C |

Die Granulate wurden nach DIN EN ISO 4577:1999 in einem Umluftofen bei 150 °C und 50 % Luftumwälzung für 80 Tage gealtert und Proben nach 5, 10 und 50 Tagen entnommen. Anschließend erfolgte die Beurteilung der Reststabilität durch statische OIT-Messung (Oxidative Induction Time) nach DIN EN ISO 11357-6:2013 mit synthetischer Luft als Spülgas bei 230 °C bzw. 210 °C. Tabelle 3 enthält die ermittelten Induktionszeiten. Dabei ist die Reststabilität des Polymeren umso höher je länger die Induktionszeit ist.

**Tabelle 3: OIT Werte nach Alterung bei 150 °C**

| | OIT [min] nach Alterung von | | | |
|---|---|---|---|---|
| | 230 °C | | | 210 °C |
| | 0 Tage | 5 Tage | 10 Tage | 50 Tage |
| Vergleichsbeispiel 1 | 3 | 0 | 0 | 0 |
| Vergleichsbeispiel 2 | 45 | 21 | 17 | 37 |
| Vergleichsbeispiel 3 | 22 | 11 | 9 | 51 |
| Vergleichsbeispiel 4 | 9 | # | 6 | 17 |
| Erfindungsgemäßes Beispiel 1 | 62 | 28 | 17 | 82 |
| Erfindungsgemäßes Beispiel 2 | 88 | 37 | 25 | 119 |

Beim unstabilisierten Polypropylen (Vergleichsbeispiel 1) erfolgt der Beginn der Oxidation in sehr kurzer Zeit, nach Alterung besteht überhaupt keine Reststabilität mehr. Die Compounds mit zugesetzten Antioxidantien, verzögerten den Beginn der Oxidation. Die erfindungsgemäßen Beispiele zeigen überraschenderweise eine überlegene Stabilität des erfindungsgemäßen Stabilisators gegenüber den Vergleichsbeispielen.

Messungen des Yellowness-Index (Spectro-guide sphere gloss mit Weißstandard, BYK Additives & Instruments) der bis zu 80 Tage gealterten und ungealterten Proben zeigten nach Tabelle 4 während des Alterungsverlaufes eine deutlich geringere Verfärbungstendenz der erfindungsgemäßen Beispiele im Gegensatz zu den Vergleichsbeispielen.

**Tabelle 4: Yellowness Index nach Alterung bei 150 °C.**

| | Zunahme Yellowness Index nach Alterung von | | |
|---|---|---|---|
| | 5 Tage | 10 Tage | 80 Tage |
| Vergleichsbeispiel 1 | 11,6 | 86,4 | Zersetzung |
| Vergleichsbeispiel 2 | 8,6 | 16,2 | 82,91 |
| Erfindungsgemäßes Beispiel 1 | 1,3 | 8,7 | 33,57 |
| Erfindungsgemäßes Beispiel 2 | 2,2 | 11,6 | 42,05 |

### b) Anwendungsprüfung des erfindungsgemäßen Beispiels C

Von den gealterten Granulaten wurde die Schmelzevolumenfließrate nach DIN EN ISO 1133-2:2011 bestimmt (2,16 kg, 230 °C).

**Tabelle 5: Zusammensetzungen der erfindungsgemäßen Beispiele und Vergleichsbeispiele**

| | |
|---|---|
| Vergleichsbeispiel 1 | Ohne Zusatz |
| Vergleichsbeispiel 2 | 0.3 % Songnox 1035 |
| Vergleichsbeispiel 4 | 0.1% Irganox 1076 / 0.2 % DLTDP |
| Erfindungsgemäßes Beispiel 3 | 0.3 % Beispiel C |

Tabelle 6 enthält die ermittelten Schmelzevolumenfließraten. Dabei ist die Reststabilität des Polymeren umso höher je niedriger die Schmelzevolumenfließrate ist.

**Tabelle 6: Schmelzevolumenfließrate nach Alterung bei 150 °C**

| | MVR [cm³ / 10 min ] nach Alterung von | | | |
|---|---|---|---|---|
| | 30 Tage | 60 Tage | 70 Tage | 80 Tage |
| Vergleichsbeispiel 1 | Nicht messbar | Nicht messbar | Nicht messbar | Nicht messbar |
| Vergleichsbeispiel 2 | 18,7 | 25,4 | Nicht messbar | Nicht messbar |
| Vergleichsbeispiel 4 | 19,4 | 20,9 | 22,7 | Nicht messbar |
| Erfindungsgemäßes Beispiel 3 | 17,9 | 19,4 | 21,3 | 22,7 |

Beim unstabilisierten Polypropylen (Vergleichsbeispiel 1) erfolgt der Kettenabbau des Polypropylens in sehr kurzer Zeit, nach Alterung besteht überhaupt keine Reststabilität mehr. Dadurch sind die MVR-Messwerte nicht mehr messbar, da ein starker Abbau und damit sehr hohe MVR-Werte (> 500) erreicht werden. Die Compounds mit zugesetzten Antioxidantien, verzögerten den Beginn des Kettenabbaus. Das erfindungsgemäße Beispiel zeigt überraschenderweise eine überlegene Stabilität des erfindungsgemäßen Stabilisators im Alterungsverlauf gegenüber den Vergleichsbeispielen.

### c) Anwendungsprüfung der erfindungsgemäßen Beispiele in Talk gefülltem Polypropylen

Analog Anwendungsprüfung des erfindungsgemäßen Beispiels A erfolgte die Beurteilung der Stabilität durch statische OIT-Messung für die erfindungsgemäßen Beispiele sowie Vergleichsbeispiele in

Tabelle 7 mit Zusatz von Talk als Füllstoff bei 230 °C ohne Alterung. Der prozentuale Gehalt an zugesetztem Stabilisator bezieht sich dabei auf den Gehalt an Polypropylen.

**Tabelle 7: Zusammensetzungen der erfindungsgemäßen Beispiele sowie Vergleichsbeispiele in Finntalc M15 gefülltem Polypropylen (alle Angaben in Gew.-%).**

| | | |
|---|---|---|
| Vergleichsbeispiel 5 | Ohne Zusatz | 20 % Finntalc M15 |
| Vergleichsbeispiel 6 | 0.1 % Irganox 1076 / 0.2 % DLTDP | 20 % Finntalc M15 |
| Erfindungsgemäßes Beispiel 4 | 0.3 % Beispiel A | 20 % Finntalc M15 |
| Erfindungsgemäßes Beispiel 5 | 0.3 % Beispiel C | 20 % Finntalc M15 |
| Erfindungsgemäßes Beispiel 6 | 0.3 % Beispiel F | 20 % Finntalc M15 |
| Nicht-erfindungsgemäßes Beispiel 7 | 0.3 % Beispiel G | 20 % Finntalc M15 |

**Tabelle 8: OIT-Werte in PP mit 20 Gew.-% Finntalc M15 vor Alterung bei 230 °C.**

| | OIT [min] |
|---|---|
| | 230 °C |
| Vergleichsbeispiel 5 | 0 |
| Vergleichsbeispiel 6 | 11 |
| Erfindungsgemäßes Beispiel 4 | 47 |
| Erfindungsgemäßes Beispiel 5 | 14 |
| Erfindungsgemäßes Beispiel 6 | 17 |
| Nicht-erfindungsgemäßes Beispiel 7 | 29 |

Beim unstabilisierten Polypropylen (Vergleichsbeispiel 5) mit dem Finntalc M15 Füllstoff, erfolgt der Beginn der Oxidation bereits direkt nach der Umschaltung von inerter Atmosphäre zu sauerstoffhaltiger Atmosphäre, so dass überhaupt keine Reststabilität mehr vorhanden ist. Die erfindungsgemäßen Beispiele zeigen eine deutlich überlegene Stabilität der erfindungsgemäßen Stabilisatoren gegenüber den Vergleichsbeispielen.

Analog wurden die erfindungsgemäßen Stabilisatoren in 10 % Talk vom Typ Luzenac 1445 nach Tabelle 9 eingearbeitet und die OIT bestimmt (Tabelle 10).

**Tabelle 9: Zusammensetzungen der erfindungsgemäßen Beispiele sowie Vergleichsbeispiele in Luzenac 1445 gefülltem Polypropylen (alle Angaben in Gew.-%).**

| | | |
|---|---|---|
| Vergleichsbeispiel 7 | Ohne Zusatz | 10 % Luzenac 1445 |
| Vergleichsbeispiel 8 | 0.1 % Irganox 1076 / 0.2 % DLTDP | 10 % Luzenac 1445 |
| Erfindungsgemäßes Beispiel 8 | 0.3 % Beispiel A | 10 % Luzenac 1445 |
| Erfindungsgemäßes Beispiel 9 | 0.2 % Beispiel A | 10 % Luzenac 1445 |
| Erfindungsgemäßes Beispiel 10 | 0.3 % Beispiel B | 10 % Luzenac 1445 |
| Erfindungsgemäßes Beispiel 11 | 0.3 % Beispiel C | 10 % Luzenac 1445 |
| Erfindungsgemäßes Beispiel 12 | 0.1 % Beispiel C | 10 % Luzenac 1445 |
| Erfindungsgemäßes Beispiel 13 | 0.3 % Beispiel F | 10 % Luzenac 1445 |
| Erfindungsgemäßes Beispiel 14 | 0.1 % Beispiel F | 10 % Luzenac 1445 |
| Nicht-erfindungsgemäßes Beispiel 15 | 0.3 % Beispiel G | 10 % Luzenac 1445 |
| Erfindungsgemäßes Beispiel 16 | 0.3 % Beispiel I | 10 % Luzenac 1445 |

**Tabelle 10: OIT-Werte In PP mit 10 wt.% Luzenac 1445 vor Alterung.**

| | OIT [min] vor Alterung |
|---|---|
| | 230 °C |
| Vergleichsbeispiel 7 | 5 |
| Vergleichsbeispiel 8 | 8 |
| Erfindungsgemäßes Beispiel 8 | 85 |
| Erfindungsgemäßes Beispiel 9 | 62 |
| Erfindungsgemäßes Beispiel 10 | 60 |
| Erfindungsgemäßes Beispiel 11 | 35 |
| Erfindungsgemäßes Beispiel 12 | 12 |
| Erfindungsgemäßes Beispiel 13 | 46 |
| Erfindungsgemäßes Beispiel 14 | 12 |
| Nicht-erfindungsgemäßes Beispiel 15 | 43 |
| Erfindungsgemäßes Beispiel 16 | 41 |

Beim unstabilisierten Polypropylen (Vergleichsbeispiel 7) mit dem Luzenac 1445 Füllstoff, erfolgt der Beginn der Oxidation bereits sehr früh. Die erfindungsgemäßen Beispiele zeigen eine deutlich überlegene Stabilität der erfindungsgemäßen Stabilisatoren gegenüber den Vergleichsbeispielen, selbst bei vergleichsweise niedrigerer Konzentration (erfindungsgemäße Beispiele 9, 12 und 14).

### d) Anwendungsprüfung der erfindungsgemäßen Beispiele in weiteren Systeme

Die erfindungsgemäße Verbindung A wurde darüber hinaus in einer Konzentration von 0.3 % in den folgenden Polymeren eingearbeitet:
D1: Acrylnitril-Butadien-Styrol (ABS)
D2: Polyamid-6
D3: Polyamid-6 mit 30 % Glasfasern
D4: Polybutylenterephthalat

In allen Fällen lässt sich gegenüber dem Polymeren ohne Zusatz eine verbesserte Langzeitstabilität, d.h. Erhalt der mechanischen Eigenschaften nachweisen.

## Patentansprüche

1. Verfahren zur Herstellung einer Verbindung gemäß der allgemeinen Formel I 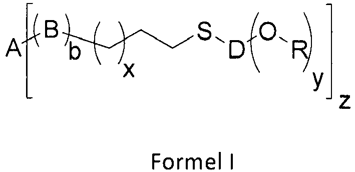 wobei die Variablen A, B, D, R, b, x, y, z jeweils unabhängig voneinander die nachfolgende Definition aufweisen:
A einen aromatischen, ungesättigten oder gesättigten Rest,
B O oder NH,
D einen linearen oder verzweigten aliphatischen Rest mit 1 bis 12 Kohlenstoffatomen,
R die Bedeutung aufweist: 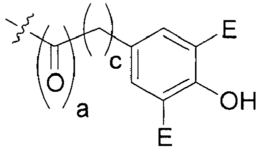 wobei
E bei jedem Auftreten gleich oder verschieden ist und einen linear aliphatischen, verzweigt aliphatischen, cycloaliphatischen Alkylrest mit 1 bis 18 Kohlenstoffatomen oder einen aromatischen Rest mit 6 bis 36 Kohlenstoffatomen darstellt,
a 1 oder 0 ist, und
c 0, 1, 2, 3, oder 4 ist,
b 0 oder 1,
x 0 bis 12,
y 1 bis 4,
z 1 bis 6,
wobei bei dem Verfahren eine Verbindung gemäß allgemeiner Formel II 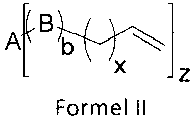 mit einem Thiol gemäß einer der allgemeinen Formeln Illa oder Illb 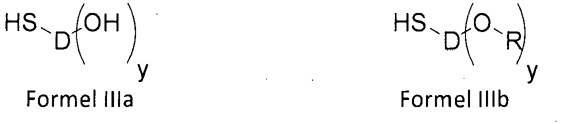 umgesetzt wird, und für den Fall, dass ein Thiol der allgemeinen Formel IIIa verwendet wurde, anschließend eine Umsetzung des durch Reaktion der Verbindungen der Formel II und IIIa erhaltenen Reaktionsproduktes mit einer Verbindung der allgemeinen Formel IV
X-R Formel IV
erfolgt, wobei
X eine Abgangsgruppe darstellt.

2. Verfahren nach vorhergehendem Anspruch, **dadurch gekennzeichnet, dass** die Variable A ausgewählt ist aus der Gruppe bestehend aus jeweils z-wertigen Cyanursäureresten, wobei z die im Anspruch 1 angegebene Bedeutung besitzt, Triazinresten, cyclischen aliphatischen Kohlenwasserstoffresten mit 5 bis 36 Kohlenstoffatomen, insbesondere Cyclohexyl, aromatischen Kohlenwasserstoffresten, insbesondere Phenyl, linearen oder verzweigten aliphatischen Kohlenwasserstoffresten mit 2 bis 36 Kohlenstoffatomen.

3. Verfahren nach einem der vorhergehenden Ansprüche, **dadurch gekennzeichnet, dass** die Variablen x, y und z jeweils unabhängig voneinander die nachfolgende Bedeutung aufweisen:
x 0 oder 1,
y 1 oder 2,
z 1, 2, 3 oder 4.

4. Verfahren nach einem der vorhergehenden Ansprüche, **dadurch gekennzeichnet, dass** die Verbindung der allgemeinen Formel I ausgewählt ist aus der Gruppe bestehend aus den nachfolgenden Verbindungen: 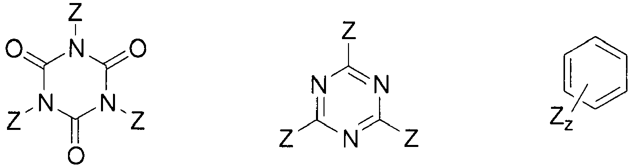 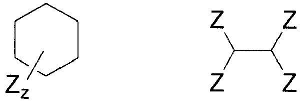 wobei Z die nachfolgende Bedeutung aufweist: 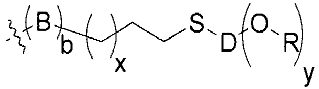 und die Variablen B, D, R, b, x, y und z wie in Anspruch 1 definiert sind.

5. Verfahren nach einem der vorhergehenden Ansprüche, **dadurch gekennzeichnet, dass** D -CH₂- ist.

6. Verfahren nach einem der vorhergehenden Ansprüche, wobei die Verbindung ausgewählt ist aus der Gruppe der nachfolgenden Verbindungen: 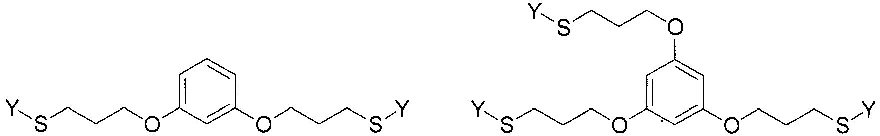 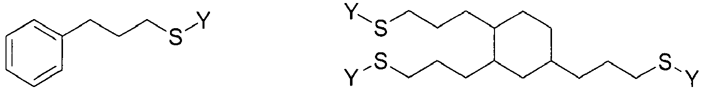 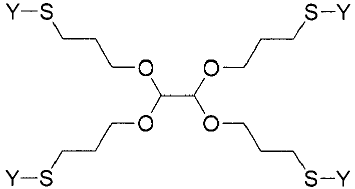 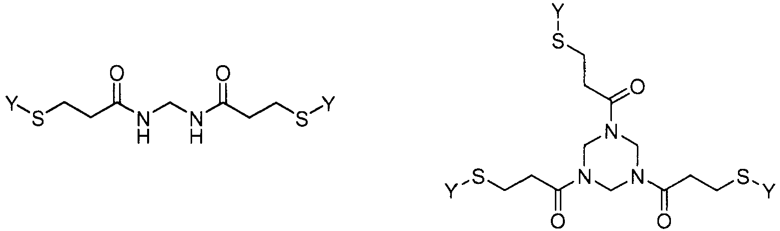 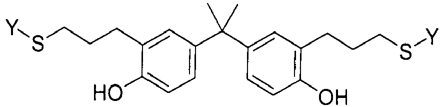 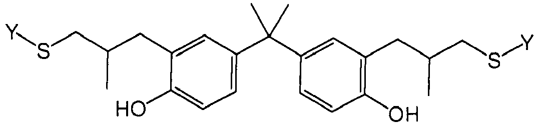 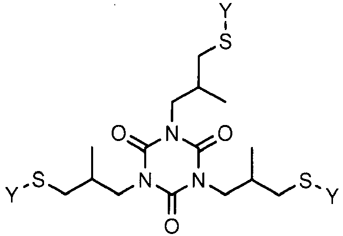 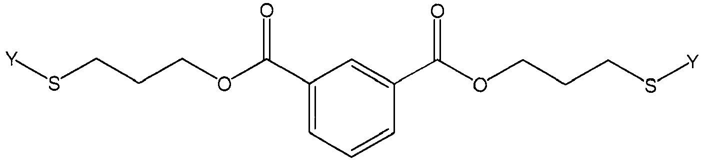 wobei bei jedem Auftreten unabhängig voneinander der Rest Y die nachfolgende Bedeutung aufweist: 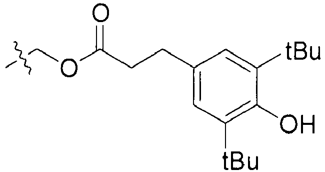 oder 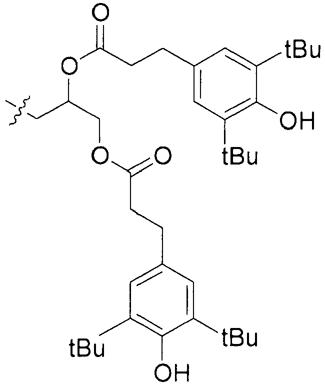 wobei in den voranstehenden Resten der tBu-Rest auch ganz oder teilweise durch eine Methylgruppe und/oder Wasserstoff substituiert sein kann.

7. Verfahren nach einem der vorhergehenden Ansprüche, **dadurch gekennzeichnet, dass** die Umsetzung der Verbindung gemäß allgemeiner Formel II mit dem Thiol gemäß einer der allgemeinen Formeln Illa oder IIIb mit einem Überschuss des Thiols, bezogen auf die ungesättigte Funktion der Verbindung gemäß allgemeiner Formel II durchgeführt wird.

8. Verbindung ausgewählt aus der Gruppe bestehend aus den nachfolgenden Verbindungen 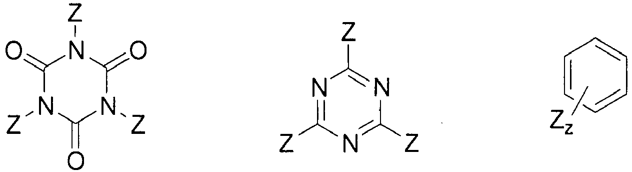 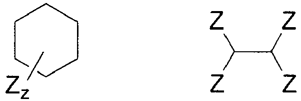 wobei Z die nachfolgende Bedeutung aufweist: 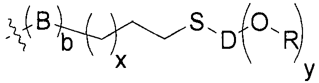 wobei die Variablen B, D, R, b, x, y, z jeweils unabhängig voneinander die nachfolgende Definition aufweisen:
B O oder NH,
D einen linearen oder verzweigten aliphatischen Rest mit 1 bis 12 Kohlenstoffatomen,
R die Bedeutung aufweist: 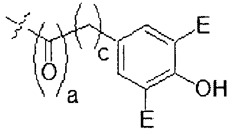
E bei jedem Auftreten gleich oder verschieden ist und einen linear aliphatischen, verzweigt aliphatischen, cycloaliphatischen Alkylrest mit 1 bis 18 Kohlenstoffatomen oder einen aromatischen Rest mit 6 bis 36 Kohlenstoffatomen darstellt,
a 1 oder 0 ist, und
c 0, 1, 2, 3, oder 4 ist,
b 0 oder 1,
x 0 bis 12,
y 1 bis 4,
z 2, 3 oder 4.

9. Zusammensetzung, enthaltend oder bestehend aus mindestens eine zu stabilisierende organische Komponente, sowie mindestens eine Verbindung nach Anspruch 8.

10. Zusammensetzung nach vorhergehendem Anspruch, **dadurch gekennzeichnet, dass** die zu stabilisierende Komponente ausgewählt ist aus der Gruppe bestehend aus Kunststoffen, Ölen, Schmiermitteln und Fetten.

11. Zusammensetzung nach einem der beiden vorhergehenden Ansprüche, enthaltend oder bestehend aus
95,00 bis 99,99 Gew.-Teile, bevorzugt 97,00 bis 99,95 Gew.-Teile, besonders bevorzugt 98,00 bis 99,90 Gew.-Teile mindestens einer zu stabilisierende Komponente, sowie
0,01 bis 5,00 Gew.-Teile, bevorzugt 0,05 bis 3,00 Gew.-Teile, besonders bevorzugt 0,10 bis 2,00 Gew.-Teile mindestens einer Verbindung nach Anspruch 8.

12. Zusammensetzung nach einem der Ansprüche 9 bis 11, **dadurch gekennzeichnet, dass** zusätzlich mindestens ein Zusatzstoff ausgewählt aus der Gruppe bestehend aus UV-Absorbern, Lichtstabilisatoren, Stabilisatoren, Antioxidantien, Hydroxylaminen, Benzofuranen, Metalldesaktivatoren, Füllstoffdesaktivatoren, Antiozonantien, Nukleirungsmitteln, Schlagzähigkeitsverbesserern, Weichmachern, Gleitmitteln, Rheologiemodifikatoren, Thixotropiemitteln, Kettenverlängerern, Verarbeitungshilfsmitteln, Entformungshilfsmitteln, Flammschutzmitteln, Pigmenten, Farbstoffen, optischen Aufhellern, antimikrobielle Wirkstoffen, Antistatika, Slipmitteln, Antiblockiermitteln, Kopplungsmitteln, Vernetzungsmitteln, Antivernetzungsmitteln, Hydrophilisierungsmitteln, Hydrophobierungsmitteln, Haftvermittlern, Dispergiermitteln, Abbau-Additiven, Entschäumungshilfsmitteln, Geruchsfängern, Markierungsmitteln, Antifoggingmitteln, Füllstoffen und Verstärkungsstoffen enthalten ist, und/oder zusätzlich mindestens ein Zusatzstoff ausgewählt aus der Gruppe bestehend aus Phosphiten, Phosphoniten, Hydroxylaminen oder Nitronen enthalten ist.

13. Verfahren zur Stabilisierung einer zu stabilisierenden organischen Komponente, insbesondere gegen oxidativen, thermischen oder actinischen Abbau oder Schädigung, bei dem mindestens eine Verbindung nach Anspruch 8 mit der zu stabilisierenden organischen Komponente vermischt oder in diese eingearbeitet wird.

14. Verwendung einer Verbindung nach Anspruch 8 zur Stabilisierung organischer Materialien gegen oxidativen, thermischen oder actinischen Abbau oder Schädigung.

## Claims

1. A method for the preparation of a compound according to the general formula I 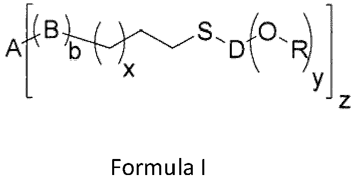 where the variables A, B, D, R, b, x, y, z each have the following definition independently of one another:
A an aromatic, unsaturated or saturated residue;
B OorNH;
D a linear or branched aliphatic residue having 1 to 12 carbon atoms;
R has the meaning: 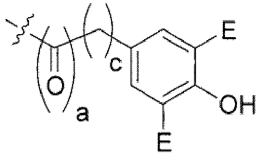 where
E is the same or different at each occurrence and is a linear aliphatic, branched aliphatic, cycloaliphatic alkyl radical having 1 to 18 carbon atoms or an aromatic radical having 6 to 36 carbon atoms,
a is 1 or 0; and
c is 0, 1, 2, 3, or 4
b 0 or 1;
x 0 to 12;
y 1 to 4;
z 1 to 6;
in which method a compound according to general formula II 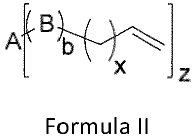 is converted with a thiol according to one of the general formulas Illa or Illb 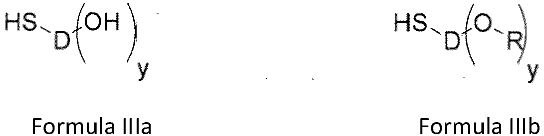 and, in the case where a thiol of the general formula Illa has been used, subsequently reacting the reaction product obtained by reacting the compounds of the formula II and Illa with a compound of the general formula IV
X-R Formula IV
where
X is a leaving group.

2. The method according to the preceding claim, **characterized in that** the variable A is selected from the group consisting of cyanuric acid radicals of z-valency in each case, wherein z has the meaning given in claim 1, triazine radicals, cyclic aliphatic hydrocarbon radicals having 5 to 36 carbon atoms, in particular cyclohexyl, aromatic hydrocarbon radicals, in particular phenyl, linear or branched aliphatic hydrocarbon radicals having 2 to 36 carbon atoms.

3. The method according to one of the preceding claims, **characterized in that** the variables x, y and z each have the following meaning independently of one another:
x 0 or 1;
y 1 or 2;
z 1, 2, 3 or 4.

4. The method according to any one of the preceding claims, **characterized in that** the compound of general formula I is selected from the group consisting of the following compounds: 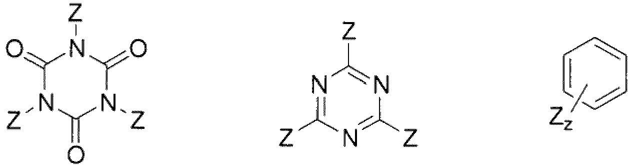 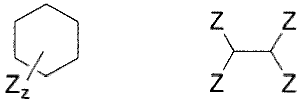 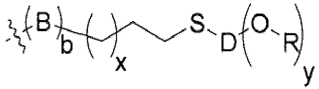 where Z has the following meaning:
and the variables B, D, R, b, x, y and z are as defined in claim 1.

5. The method according to any one of the preceding claims, **characterized in that** D is -CH₂₋.

6. The method of any one of the preceding claims, wherein the compound is selected from the group consisting of the following compounds: 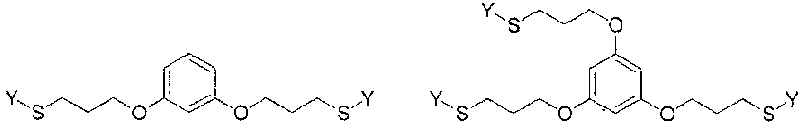 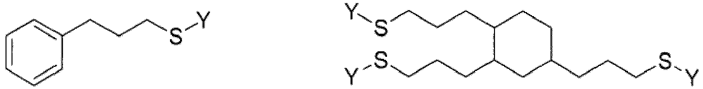 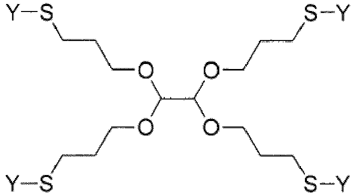 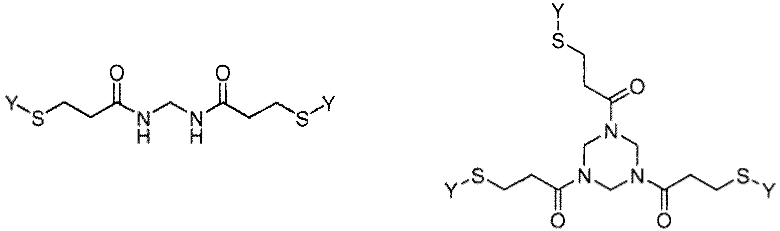 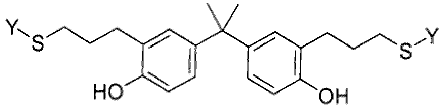 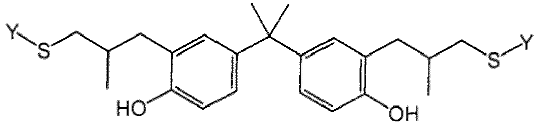 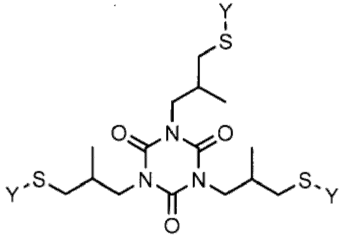 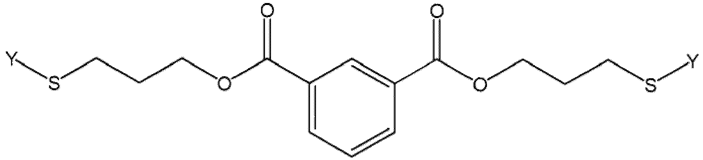 where, in each occurrence independently, the residue Y has the following meaning: 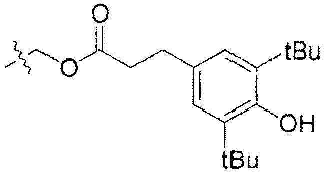 or 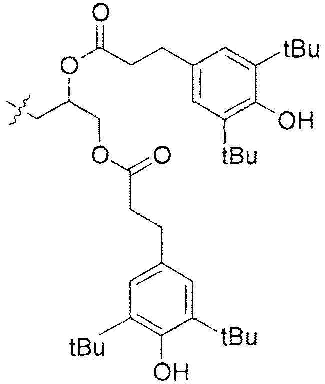 wherein, in the preceding radicals, the tBu radical may also be wholly or partially substituted by a methyl group and/or hydrogen.

7. The method according to any one of the preceding claims, **characterized in that** the reaction of the compound according to general formula II with the thiol according to one of the general formulas Illa or Illb is carried out with an excess of the thiol relative to the unsaturated function of the compound according to general formula II.

8. A compound selected from the group consisting of the following compounds 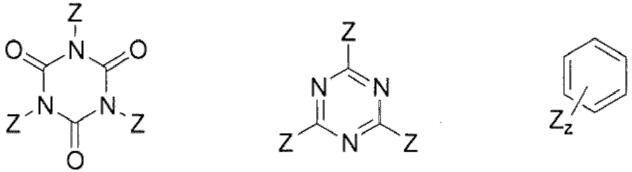 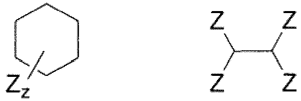 where Z has the following meaning: 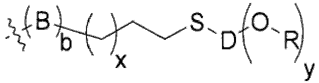 where the variables B, D, R, b, x, y, z each independently have the following definition:
B OorNH;
D a linear or branched aliphatic residue having 1 to 12 carbon atoms;
R has the meaning: 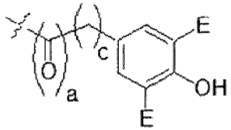
E is the same or different at each occurrence and is a linear aliphatic, branched aliphatic, cycloaliphatic alkyl radical having 1 to 18 carbon atoms or an aromatic radical having 6 to
36 carbon atoms,
a is 1 or 0; and
c is 0, 1, 2, 3, or 4;
b 0 or 1;
x 0 to 12;
y 1 to 4;
z 2, 3 or 4.

9. A composition comprising or consisting of at least one organic component to be stabilized, and at least one compound according to claim 8.

10. A composition in accordance with the preceding claim, **characterized in that** the component to be stabilized is selected from the group consisting of plastics, oils, lubricants, and fats.

11. A composition in accordance with one of the two preceding claims, comprising or consisting of
95.00 to 99.99 wt%, preferably 97.00 to 99.95 wt%, particularly preferably 98.00 to 99.90 wt%, of at least one component to be stabilized; and
0.01 to 5.00 wt%, preferably 0.05 to 3.00 wt%, particularly preferably 0.10 to 2.00 wt%, of at least one compound in accordance with claim 8.

12. A composition in accordance with one of the claims 9 to 11, **characterized in that** additionally at least one additive is selected from the group comprising UV absorbers, light stabilizers, stabilizers, antioxidants, hydroxyl amines, benzofurans, metal deactivators, filler deactivators, antiozonants, nucleation agents, impact strength improvers, plasticizers, lubricants, rheology modifiers, thixotropic agents, chain extenders, processing aids, demolding aids, flame retardants, pigments, dyes, optical brighteners, antimicrobial agents, antistatic agents, slip agents, antiblocking agents, coupling agents, crosslinking agents, anti-crosslinking agents, hydrophilization agents, hydrophobization agents, bonding agents, dispersion agents, degradation additives, defoaming aids, odor traps, marking agents, antifogging agents, fillers, and reinforcements, and/or additionally at least one additive selected from the group consisting of phosphites, phosphonites, hydroxylamines or nitrones is present.

13. A method of stabilizing an organic component to be stabilized, in particular with respect to oxidative, thermal, or actinic degradation or damage, in which at least one compound in accordance with claim 8 is mixed with the organic component to be stabilized or is worked into it.

14. Use of a compound in accordance with claim 8 for stabilizing organic materials with respect to oxidative, thermal, or actinic degradation or damage.

## Revendications

1. Procédé de fabrication d'un composé selon la formule générale I 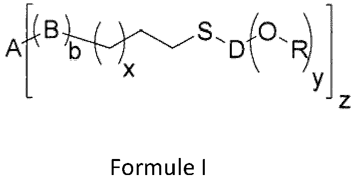 dans laquelle les variables A, B, D, R, b, x, y, z présentent chacune indépendamment entre elles la définition suivante :
A : un résidu aromatique, non saturé ou saturé,
B: OouNH,
D : un résidu aliphatique linéaire ou ramifié avec 1 à 12 atomes de carbone,
R : présente la signification : 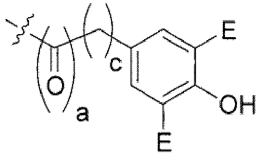
dans laquelle
E : est le même ou différent à chaque apparition et représente un résidu alkyle aliphatique linéaire, aliphatique ramifié, cyclo-aliphatique, avec 1 à 18 atomes de carbone ou un résidu aromatique avec 6 à 36 atomes de carbone,
a : est égale à 1 ou 0 et
c : est égale à 0, 1, 2, 3 ou 4,
b : est égal à 0 ou 1,
x : 0 à 12,
y : est de 1 à 4,
z : est de 1 à 6,
dans lequel, dans le procédé, un composé selon la formule générale II 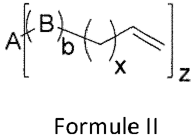 avec un thiol selon l'une des formules générales IIIa ou IIIb 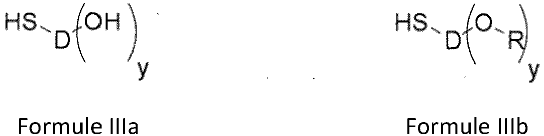 est mis en oeuvre et, dans le cas où un thiol de formule générale Illa a été utilisé, une mise en oeuvre du produit de réaction obtenu grâce à la réaction des composés de Formules II et IIIa a lieu avec un composé de formule générale IV
X-R Formule IV
dans lequel
X représente un groupe partant.

2. Procédé selon la revendication précédente, **caractérisé en ce que** la variable A est sélectionnée dans le groupe constitué respectivement de résidus d'acide cyanurique z-valent, dans lequel z présente la signification indiquée dans la revendication 1, des résidus de triazine, de résidus d'hydrocarbures aliphatiques cycliques avec 5 à 36 atomes de carbone, plus particulièrement du cyclohexyle, de résidus d'hydrocarbures aromatiques, plus particulièrement du phényle, des résidus d'hydrocarbures aliphatiques linéaires ou ramifiés avec 2 à 36 atomes de carbone.

3. Procédé selon l'une des revendications précédentes, **caractérisé en ce que** les variables x, y et z présentent chacune, indépendamment entre elles, la signification suivante :
x : 0 ou 1,
y : 1 ou 2,
z : 1, 2, 3 ou 4.

4. Procédé selon l'une des revendications précédentes, **caractérisé en ce que** le composé de formule générale I est sélectionné dans le groupe constitué des composés suivants : 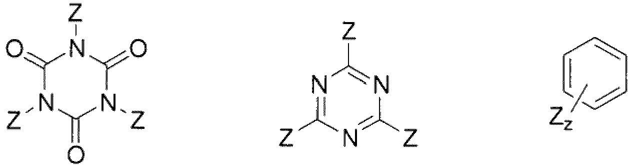 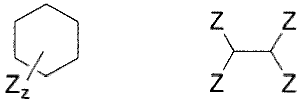 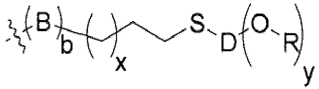 dans lequel Z présente la signification suivante :
et les variables B, D, R, b, x, y et z sont définies comme dans la revendication 1.

5. Procédé selon l'une des revendications précédentes, **caractérisé en ce que** D est -CH₂-.

6. Procédé selon l'une des revendications précédentes, **caractérisé en ce que** le composé est sélectionné dans le groupe de composés suivants : 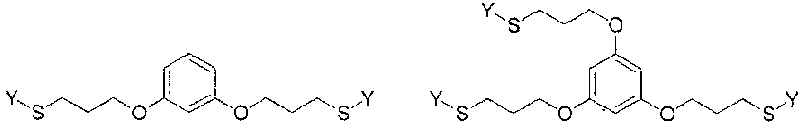 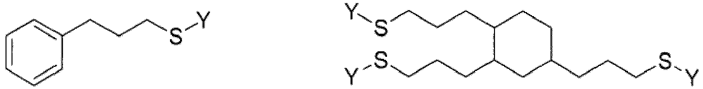 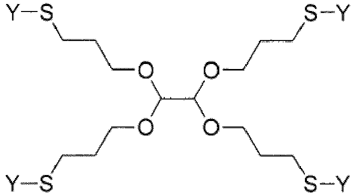 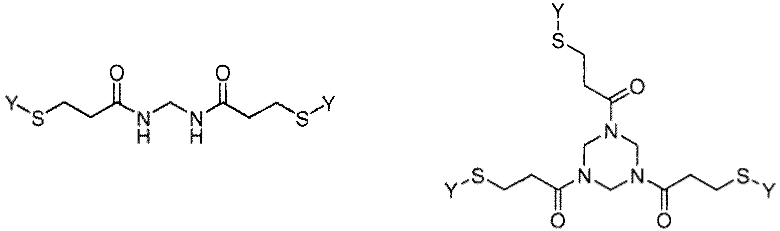 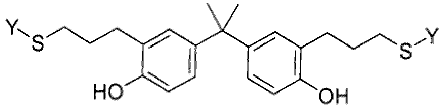 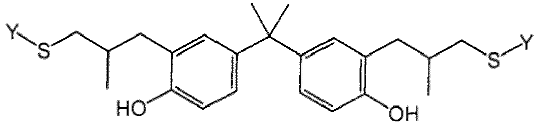 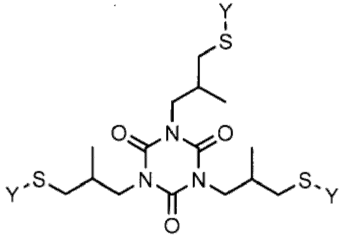 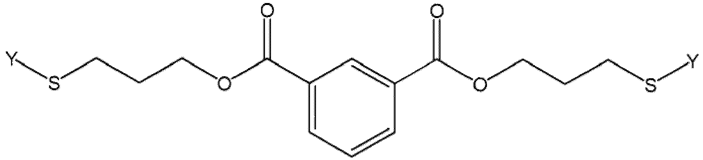 dans lequel, à chaque apparition, indépendamment entre elles, le résidu Y présente la signification suivante : 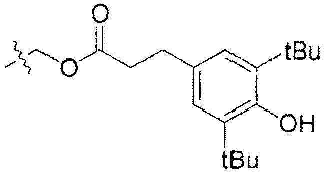 ou 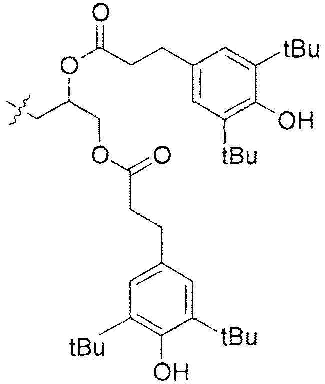 dans lequel, dans les résidus précédents, le résidu tBu peut également être substitué entièrement ou partiellement par un groupe méthyle et/ou un hydrogène.

7. Procédé selon l'une des revendications précédentes, **caractérisé en ce que** la mise en oeuvre du composé selon la formule générale II avec le thiol selon l'une des formules générales Illa ou IIIb est effectuée avec un excès de thiol par rapport à la fonction insaturée du composé selon la formule générale II.

8. Composé sélectionné dans le groupe constitué des composés suivants 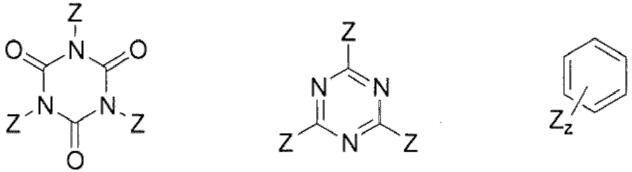 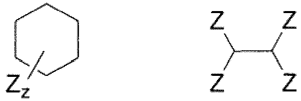 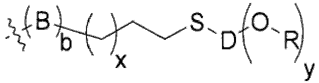 dans lequel Z présente la signification suivante :
dans lequel les variables B, D, R, x, y, z présentent chacune, indépendamment entre elles, la définition suivante :
B: Oou NH,
D : un résidu aliphatique linéaire ou ramifié avec 1 à 12 atomes de carbone,
R : présente la signification : 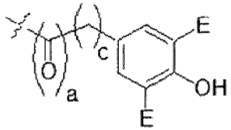
E est le même ou différent à chaque apparition et représente un résidu alkyle aliphatique linéaire, aliphatique ramifié, cyclo-aliphatique avec 1 à 18 atomes de carbone ou un résidu aromatique avec 6 à 36 atomes de carbone,
a : est 1 ou 0 et
c : est 0, 1, 2, 3 ou 4,
b : 0 ou 1,
x : 0 à 12,
y : 1 à 4,
z : 2, 3 ou 4.

9. Composition contenant ou constituée d'au moins un composant organique à stabiliser, ainsi qu'au moins un composé selon la revendication 8.

10. Composition selon la revendication précédente, **caractérisée en ce que** le composant à stabiliser est sélectionné dans le groupe constitué de matières plastiques, d'huiles, de lubrifiants et de graisses.

11. Composition selon l'une des deux revendications précédentes contenant ou constituée de
95,00 à 99,99 parties en poids, de préférence 97,00 à 99,95 parties en poids, plus particulièrement de préférence 98,00 à 99,90 parties en poids d'au moins un composant à stabiliser, ainsi que de
0,01 à 5,00 parties en poids, de préférence 0,05 à 3,00 parties en poids, plus particulièrement de préférence 0,10 à 2,00 parties en poids d'au moins un composé selon la revendication 8.

12. Composition selon l'une des revendications 9 à 11, **caractérisée en ce que**, en outre, au moins un additif, sélectionné dans le groupe constitué d'agents absorbant les UV, de photo-stabilisateurs, de stabilisateurs, d'antioxydants, d'hydroxylamines, de benzofuranes, de désactivateurs de métaux, de désactivateurs de charges, d'antiozonants, d'agents de nucléation, d'agents améliorant la résistance aux chocs, d'agents émollients, d'agents lubrifiants, de modificateurs de rhéologie, d'agents de thixotropie, d'agents d'allongement de chaînes, d'auxiliaires de traitement, d'auxiliaires de déformation, d'agents ignifuges, de pigments, de colorants, d'éclaircissants optiques, d'agents actifs antimicrobiens, d'agents antistatiques, d'agents de glissement, d'agents anti-blocage, d'agents de couplage, d'agents de réticulation, d'agents anti-réticulation, d'agents d'hydrophilisation, d'agents d'hydrophobisation, de promoteurs d'adhérence, d'agents dispersants, d'additifs de décomposition, d'auxiliaires anti-mousse, d'agents captant les odeurs, d'agents de marquage, d'agents anti-brouillard, de charges et de substances de renforcement, est contenu, et/ou, en outre, au moins un additif sélectionné dans le groupe constitué de phosphites, de phosphonites, d'hydroxylamines ou de nitrones.

13. Procédé de stabilisation d'un composant organique à stabiliser, plus particulièrement contre une décomposition ou une dégradation oxydative, thermique ou actinique, dans lequel au moins un composé selon la revendication 8 est mélangé avec le composant organique à stabiliser ou intégré dans celui-ci.

14. Utilisation d'un composé selon la revendication 8 pour la stabilisation de matériaux organiques contre une décomposition ou une dégradation oxydative, thermique ou actinique.
